# EUROPEAN PATENT APPLICATION

(11) **EP 4 386 770 A1**
(43) Date of publication of application: **19.06.2024**
(21) Application number: 22855919.1
(22) Date of filing: 12.08.2022
(51) Int. Cl.: G16H 20/00

(54) **INFORMATION PROVISION METHOD FOR PREDICTING HEALTH CONDITION OF CONSUMER AND SUPPORTING HEALTH MAINTENANCE AND IMPROVEMENT**

(30) Priority: 13.08.2021 JP 2021131860; 04.08.2022 JP 2022125041
(71) Applicant: Otsuka Pharmaceutical Co., Ltd., Tokyo 101-8535 (JP)
(72) Inventor: NAKAGAWA, Yasushi, Osaka-shi, Osaka 540-0021 (JP); DEGUCHI, Naoyuki, Osaka-shi, Osaka 540-0021 (JP); HAMAMOTO, Keisuke, Osaka-shi, Osaka 540-0021 (JP); TOBA, Masamichi, Osaka-shi, Osaka 540-0021 (JP); IWASHITA, Soh, Osaka-shi, Osaka 540-0021 (JP); TSUBOUCHI, Mina, Osaka-shi, Osaka 540-0021 (JP); KWAK, Kyungtak, Osaka-shi, Osaka 540-0021 (JP); YOSHIE, Miho, Osaka-shi, Osaka 540-0021 (JP)
(74) Representative: Hoffmann Eitle
(86) International application number: PCT/JP2022/030726
(87) International publication number: WO 2023/017857

(57) **Abstract**

A technique capable of calling attention for a subject to a predetermined disease, a disorder that does not correspond to a disease but that is accompanied by a symptom obstructing the subject's daily life, and the like, is provided.

An information providing method includes acquiring personal data of a target customer, determining a segmentation to which the target customer belongs based on the personal data and a classification criterion that is prepared in advance, from plural segmentations each indicating a class that corresponds to at least the degree of a health condition, and providing health information that corresponds to the determined segmentation to the target customer. The personal data includes a plurality of pieces of unique data on the target customer. At least one of the plurality of pieces of unique data is symptom data that reflects a health symptom of the target customer. The plural segmentations are classified corresponding to the degrees of the health symptom, and each of the segmentations is correlated with health information prepared in advance corresponding to the health symptom.

## Description

### TECHNICAL FIELD

The present disclosure relates to an information providing method to predict the health condition of a person in an ordinary life and to support maintenance and improvement of the health of the person.

### BACKGROUND ART

The life environment has recently been drastically changing throughout the world such as the depopulation, the economic depression, occurrence of natural disasters, and prevalence of infectious diseases. On the other hand, opportunities to actively use artificial intelligence (AI) are increasing, and development and prevalence of the fifth generation mobile communication system, and the like are advancing. In this change of the life environment, the infrastructures supporting the general society start to transition to an industrial frame in which the infrastructures actively use technologies and link to each other based on data, and visualization of information on the social activities and behaviors has started.

With the growing interest in health, it is also required to adapt the efforts for health management to the social movement. The "passive healthcare" has been predominant so far with which a person receives medical treatment after the person has been affected with a disease and the person's symptomatic worsening has started. It is, however, considered that such medical cares will also be conducted from now on, as "proactive medical care" with which each healthy person acquires pieces of data on the person at home and the pieces of data are collected to present the health condition of the person as data to thereby conduct look-ahead health management and "preventive medical care" to conduct disease prevention. It can also be assumed that these medical cares will be followed by "predictive medical care" to conduct prediction of future diseases and calling attention to health.

Patent Document 1 discloses a technique according to which a personal health record (PHR) including the genome information of each user is accumulated in a large-scale genome cohort database and a cohort analysis is conducted for this PHR big data. With the technique of Patent Document 1, the correlation is derived between a combination of the genome type and the lifestyle type, and a health risk (that is, a disease onset risk), based on the result of the analysis, and the future health risk of a user is estimated.

### PRIOR ART DOCUMENT

### PATENT DOCUMENT

Patent Document 1: JP2017006745A

### SUMMARY OF INVENTION

### PROBLEMS TO BE SOLVED BY INVENTION

Various methods can be considered for providing information on the health risk. When the information providing method is diversified, a user tends to easily acquire the information needed by the user. A new method for providing information on the health risk is required.

One object of the present invention is to provide a technique capable of calling attention for a subject to a predetermined disease, a disorder that does not correspond to a disease but that is accompanied by a symptom obstructing the subject's daily life, or the like.

### SOLUTIONS TO PROBLEMS

The invention of the present application provides the following:
[1]An information providing method includes the steps of acquiring personal data of a target customer, determining a segmentation to which the target customer belongs based on the personal data and a classification criterion prepared in advance from plural segmentations each indicating a class that corresponds to a degree of a health condition, and providing health information corresponding to the determined segmentation, to the target customer. The personal data includes a plurality of pieces of unique data on the target customer, and at least one of the plurality of pieces of unique data is health data or behavior data that reflects a health symptom of the target customer. The plural segmentations are each classified corresponding to a degree of the health symptom, and each of the segmentations is correlated with health information prepared in advance corresponding to the health condition.
[2] The information providing method described in [1], in which
   the degree of the health condition is classified in plural numerical ranges, in which
   each of the plural segmentations is correlated in advance with a numerical range that corresponds to the degree of the health condition, in which
   the degree of a potential health condition of the target customer is indicated by a numerical value, and in which
   the degree of the potential health condition of the target customer is predicted by determining a segmentation that corresponds to the numerical value, based on the numerical range to which the numerical value belongs.
[3] The information providing method described in [2], in which
   the plural segmentations are three or more segmentations.
[4] The information providing method described in [2] or [3], in which
   a table is prepared in advance, that correlates therein each of the plural segmentations, and a service and/or a product that suits the health condition of each of the segmentations with each other, and in which
   a proposal relating to the service and/or the product that suits the health condition in the determined class, together with the health information, is further provided.
[5] The information providing method described in [4], in which
   the health information and/or the proposal are/is provided through a text message in an e-mail, a display on a displaying device, printing on a receipt, or a message presented on an app executed by an electronic device.
[6] The information providing method described in any one of [1] to [5], in which
   in an aspect, the potential health condition of the target customer is one or more selected from a health problem of a woman in her old age, a climacteric symptom, and a premenstrual syndrome or a premenstrual dysphoric disorder (PMS, PMDD) symptom.
[7] The information providing method described in any one of [1] to [5], in which
   the potential health condition of the target customer is one or more selected from a health problem caused by an unfavorable sleeping condition determined in advance, worsening of the sleeping condition, unsteadiness of the sleep rhythm, and an improper sleeping time.
[8] The information providing method described in any one of [1] to [5], in which
   the potential health condition of the target customer is one or more selected from a change of the physical condition and a lack of exercise accompanied by a lifestyle-related disease and/or obesity, obesity, and a variation of the body weight in a specific time period.
[9] The information providing method described in any one of [1] to [5], in which
   potential health information of the target customer is one or more selected from a health condition caused by water shortage, a high value of serum Na, and a high value of the BUN/creatinine ratio.
[10] The information providing method described in any one of [1] to [5], in which
   the potential health information of the target customer is one or more selected from a health problem caused by a weakened immunity, tendency to catch a cold, the AIgA concentration, an allergy symptom, and the condition of the oral cavity health.
[11] The information providing method described in any one of [1] to [5], in which
   the potential health information of the target customer is one or more selected from a health problem relating to nutrition, poor nutrition, a nutritional bias, and the dietary habit.
[12] The information providing method described in [1], in which
   the target customer is a woman, and in which
   the plurality of pieces of unique data on the target customer include
   the age of the target customer; and
   a value indicating the ability for equol production of the target customer as the health data.
[13]The information providing method described in [12], in which
   the plural segmentations are classified corresponding to combinations each of plural life stages each determined corresponding to the age, and presence or absence of the ability for equol production, and in which
   each of the segmentations is correlated with health information prepared in advance.
[14] The information providing method described in [13], in which
   the presence or the absence of the ability for equol production is determined based on a relation between a value indicating the ability for equol production and a threshold value.
[15] The information providing method described in [1], in which
   the plurality of pieces of unique data on the target customer include
   the age, the sex, the body height, the body weight, and a variation of the body weight in a specific time period, of the target customer, and
   presence or absence of a smoking habit and behavior data on the number of steps, of the target customer as health data.
[16] The information providing method described in [15], in which
   the plural segmentations are classified corresponding to combinations each of the age, the sex, the body-mass index, a variation of the body weight in a specific time period, and the behavior data on the number of steps, and the presence or absence of a smoking habit, and in which
   each of the segmentations is correlated with health information prepared in advance.
[17] The information providing method described in [1], in which
   the plurality of pieces of unique data on the target customer include
   the insomnia score, and the time period value indicating the sleeping time, of the target customer, and
   the time period value indicating a social jet lag of the target customer as symptom data.
[18] The information providing method described in [17], in which
   the plural segmentations are classified corresponding to combinations each of the degree of insomnia, and presence or absence of the social jet lag, and in which
   each of the segmentations is correlated with health information prepared in advance corresponding to the degree of a problem relating to sleeping as the health condition.
[19] The information providing method described in [1], in which
   the plurality of pieces of unique data on the target customer include
   the age of the target customer and
   the serum Na value and the BUN/creatinine ratio of the target customer or
   the serum Na value, and the urine specific gravity and/or the urine color of the target customer, as the health data.
[20] The information providing method described in [19], in which
   the plural segmentations are classified corresponding to combinations each of the serum Na value and being equal to or older than, or younger than a predetermined age, and in which
   each of the segmentations is correlated with health information prepared in advance corresponding to the degree of a problem relating to a water amount shortage as a health condition.
[21]The information providing method described in [1], in which
   the plurality of pieces of unique data on the target customer include
   the ideal body weight that the target customer regards as being ideal and
   the age, the body height, and the body weight of the target customer, as the health data.
[22] The information providing method described in [21], in which
   the plural segmentations are classified corresponding to combinations each of the age group determined by the age, the current body-mass index, and an ideal body-mass index calculated from the body height and the ideal body weight, and in which
   each of the segmentations is correlated with health information prepared in advance corresponding to the degree of a problem relating to nutrition as the health condition.
[23] The information providing method described in [1], in which
   the plurality of pieces of unique data on the target customer include salivary IgA.
[24] The information providing method described in [23], in which
   the plural segmentations are classified corresponding to the value of the salivary IgA, and in which
   each of the segmentations is correlated with health information prepared in advance corresponding to the degree of a problem relating to the immunity as the health condition.
[25] The information providing method described in any one of [1] to [24], in which
   the plural segmentations further include classes that each correspond to a daily behavior, and in which
   the plurality of pieces of unique data included in the personal data further include health data that reflects the health condition of the target customer.
[26] An information providing method including the steps of
   preparing in advance plural contents to be presented to a customer and provision order information that defines order of providing the plural contents corresponding to the degree of a health risk of the customer,
   acquiring risk data that indicates the degree of the health risk of a target customer, and
   providing the plural contents in the order that corresponds to the degree of the health risk of the target customer based on the risk data and the provision order information.
[27] The information providing method described in [26], in which
   the risk data is
   data indicating a segmentation selected from plural segmentations each representing a class that corresponds to at least the degree of a health condition based on personal data of the target customer and a classification criterion that is prepared in advance, in which
   the personal data includes a plurality of pieces of unique data on the target customer, in which
   at least one of the plurality of pieces of unique data is health data that reflects a health condition of the target customer, and in which
   the plural segmentations are classified corresponding to the degree of the health condition, and
   each of the segmentations is correlated with health information prepared in advance corresponding to the health condition.

### EFFECTS OF INVENTION

According to an exemplary embodiment of the present invention, a technique can be provided, that is capable of calling attention for a subject to a predetermined disease, a disorder that does not correspond to a disease but that is accompanied by a symptom obstructing the subject's daily life, or the like.

### BRIEF DESCRIPTION OF DRAWINGS

Fig. 1 is a hardware configuration diagram of an information processing device according to an exemplary embodiment of the present invention.
Fig. 2 is a diagram showing an example of a display used in the case where health information on the health of a woman is provided.
Fig. 3 is a diagram showing an example of a classification criterion to classify presence or absence of the ability for equol production.
Fig. 4 is a diagram showing a table that correlates therein segmentations relating to the health of a woman and file names with each other.
Fig. 5 is a diagram showing an example of a display used in the case where health information on the life style is provided.
Fig. 6 is a diagram showing an example of a display used in the case where health information on sleeping is provided.
Fig. 7 is a diagram showing a table that correlates therein segmentations relating to the sleeping and file names with each other.
Fig. 8 is a diagram showing an example of a display used in the case where health information on a water amount in the body is provided.
Fig. 9 is a diagram showing a relation among segmentations corresponding to input items, prediction results as to whether the water amount shortage is present or absent, and predicted items of health risks caused by the water amount shortage, presented when the serum Na value, the urine specific gravity, and the urine color are input.
Fig. 10 is a diagram showing a first example of a display of health information as to the health risk.
Fig. 11 is a diagram showing a second example of the display of the health information as to the health risk.
Fig. 12 is a diagram showing a third example of the display of the health information as to the health risk.
Fig. 13 is a diagram showing an example of a display used in the case where health information on nutrition is provided.
Fig. 14 is diagram showing one example of the health information to be provided to a customer in a segmentation 1.
Fig. 15 is a diagram showing one example of the health information to be provided to a customer in a segmentation 2.
Fig. 16 is a diagram showing one example of the health information to be provided to a customer in a segmentation 3.
Fig. 17 is a diagram showing one example of the health information to be provided to a customer in a segmentation 4.
Fig. 18 is a diagram showing one example of the health information to be provided to a customer in a segmentation 5.
Fig. 19 is a diagram showing one example of the health information to be provided to a customer in a segmentation 6.
Fig. 20 is a diagram showing a segmentation that corresponds to the value of the salivary IgA, presented when the salivary IgA is input.
Fig. 21 is a diagram showing one example of the health information to be provided to a customer in a segmentation at a level 1.
Fig. 22 is a diagram showing one example of the health information to be provided to a customer in a segmentation at a level 2.
Fig. 23 is a diagram showing one example of the health information to be provided to a customer in a segmentation at a level 3.
Fig. 24 is a diagram showing the details to execute health feedback and to make a proposal for a product to improve the health condition, for each of various health conditions.
Fig. 25A is a diagram showing another exemplification of the health condition and an example of the unique data to be input by a customer for each of the health conditions.
Fig. 25B is a diagram showing another exemplification of the health condition and an example of the unique data to be input by the customer for each of the health conditions.
Fig. 25C is a diagram showing another exemplification of the health condition and an example of the unique data to be input by the customer for each of the health conditions.
Fig. 26 is a flowchart showing a procedure for a process according to the exemplary embodiment of the present invention.
Fig. 27A is a schematic diagram showing various pieces of information and opportunities to be received by the customer.
Fig. 27B is a diagram showing one example of services to be provided to the customer.
Fig. 28 is a block diagram showing the configuration of an information providing system according to the second embodiment of the present disclosure.
Fig. 29 is a block diagram showing the configuration of an information terminal.
Fig. 30 is a diagram showing an example where a CPU of a server device changes information to be transmitted to the information terminal corresponding to the degree of the sleeping risk.
Fig. 31 is a diagram showing a table that summarizes the result of a questionnaire.
Fig. 32 is a transition diagram of the display content showing that the order of the pieces of information to be provided is changed depending on "slight", "intermediate", or "severe" of the sleeping risk.
Fig. 33 is a diagram exemplifying the relation between the reason for the case where each of users A to E considers that the user desires to continuously purchase merchandise, and a factor influencing the reason (presented information).
Fig. 34 is a flowchart showing a procedure for a process according to the second embodiment.

### DESCRIPTION OF EMBODIMENTS

Exemplary embodiments of the present invention will be described below in detail with reference to the accompanying drawings. No description that is more detailed than is necessary may be made. For example, no detailed description of an already well known item and no redundant description for a substantially identical configurations may be made. This is to avoid making the following description unnecessarily redundant and to facilitate the understanding by those skilled in the art. The inventor provides the accompanying drawings and the following description for those skilled in the art to fully understand the present disclosure, and these are not intended to limit thereto the subject matter described in the claims.

The configurations and the operations of the embodiments described below are exemplification. The present disclosure is not limited to the configurations and the operations of the embodiments described below. The drawings are schematic drawings and are not necessarily those that are strictly shown. In the drawings, substantially identical configurations are denoted by the same reference numerals, and redundant description therefor will not be made or will be simplified.

The present disclosure relates to a providing method for health information. In the following, an aspect will be described in which health information is provided using an information processing device as one example.

### (First Embodiment)

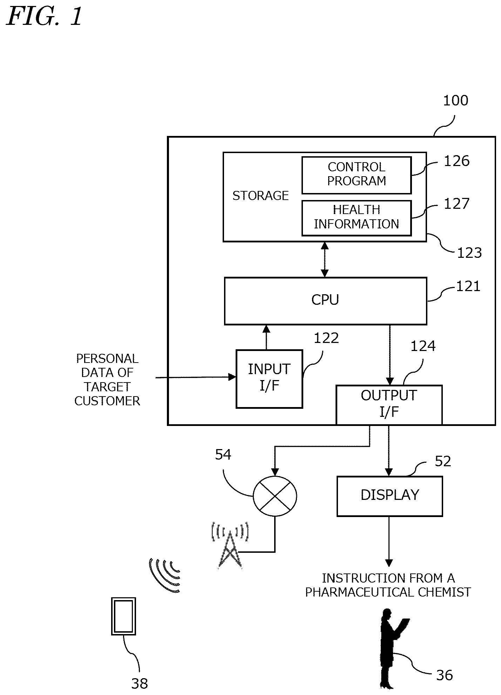

Fig. 1 is a hardware configuration diagram of an information processing device 100 used relating to this embodiment. The information processing device 100 receives personal data of a target customer, and provides health information that corresponds to the health condition of the target customer using the personal data. The target customer is an optional person who does not know so far the target customer's health risk. Otherwise, the target customer may be a person who is evaluated to potentially have a health risk based on, for example, the result of a questionnaire research already executed, the result of a medical examination, information provision from a medical examination institution, or the like. According to the above, for the target customer recognized to have a risk as a result of each of various methods, the health information corresponding to the health condition can be categorized more accurately and/or more specifically.

The information processing device 100 can be, for example, a PC or a server computer. For example, the information processing device 100 includes a central processing unit (CPU) 121, an input interface (I/F) 122, a storage 123, and an output interface (I/F) 124.

The CPU 121 is one example of a computing circuit of the information processing device in this embodiment. The CPU 121 realizes the operation described later, by executing a control program 126 stored in the storage 123. The CPU 121 executes the control program 126, and the function as the information processing device 100 of this embodiment is thereby realized. The computing circuit constituted as the CPU 121 in this embodiment may be realized by one of various types of processor such as an MPU or a GPU.

The input I/F 122 is an input device to accept an input of the personal data of the target customer. For example, the input I/F 122 may be a communication circuit or may be a touch panel, a keyboard, or a mouse.

The storage 123 is a storage medium that stores therein a computer program 126 necessary for operating the information processing device 100 and health information 127. The storage 123 can be, for example, a hard disc drive (HDD) or a solid state drive (SSD) that is a semiconductor storage. The storage 123 may include a temporary storing element that includes a RAM such as, for example, a DRAM or an SRAM, or may function also as a working area for the CPU 121. The control program 126 is one example of the computer program in this embodiment. The details of the health information 127 will be described later.

The output I/F 124 is a communication circuit and/or a communication terminal connected to various types of output device disposed outside the information processing device 100. For example, the output I/F 124 is a video output terminal connected to a display 52. The CPU 121 of the information processing device 100 transmits the information to be provided to a customer, to the display 52 to cause the display 52 to display the information thereon. A pharmaceutical chemist 36 recognizes the content displayed on the display 52 and proposes, for example, one or plural piece(s) of merchandise purchased by many of the subjects each having a climacteric symptom, to a customer having a climacteric symptom. Otherwise, the customer recognizes the content displayed on the display 52 and studies on the merchandise and/or the services that are proposed.

Otherwise, the output I/F 124 transmits the health information to, for example, a smartphone 38 of the customer through a mobile phone line. In the case where the input I/F 122 and the output I/F 124 are each a communication terminal or a communication circuit that is connected to a communication network 54 or the like and that cam execute data communication, the hardware of the output I/F 124 and that of the input I/F 122 may be identical to each other. At this time, the input I/F 122 and the output I/F 124 can be collectively referred to as "communication interface (I/F)". The communication I/F is, for example, communication terminals of Ethernet (a registered trademark), or USB (a registered trademark) terminals. Otherwise, the communication I/F is communication circuits that each execute communication complying with a standard such as IEEE 802.11, 4G, or 5G. The communication I/F may be capable of being connected to a communication network such as an intranet or the Internet. The information processing device 100 may execute direct communication with another device through the communication I/F, or may execute communication through an access point.

The operation of the information processing device 100 will be described below.

The input I/F 122 acquires the personal data of the target customer. The "personal data" is the data unique to the target customer (hereinafter, described as "unique data"), and is, for example, the age, the sex, the body height, and the body weight. Furthermore, in this embodiment, the unique data includes health data that reflects the health condition of the target customer, and behavior data. One example of the health data is the ability for equol production, a smoking habit, the serum Na value, or the BUN/creatinine ratio, and the behavior data is the time period that indicates the "social jet lag" that is unsteadiness of the rhythm in the body and/or the number of steps. In this embodiment, it is assumed that the personal data includes a plurality of pieces of unique data and it is further assumed that at least one of the plurality of pieces of unique data is the health data or the behavior data.

The CPU 121 determines the segmentation to which the target customer belongs from plural segmentations, based on the personal data and a classification criterion prepared in advance. Each of the "plural segmentations" is a class that corresponds to the degree of the health condition. The plural segmentations can be two, or three or more to be provided. The specific example of the segmentations will be described later.

The health information 127 stored in advance in the storage 123 is a set of descriptions each for one of the various health conditions that are assumed for a user. The health information 127 includes such plural descriptions as, for example, a description on medical findings, a description for an expected symptom, and a description presenting advice to mitigate a symptom, corresponding to the degree of the health condition.

Each of the segmentations and the health information 127 are correlated with each other in advance corresponding to the health condition. When the CPU 121 determines a segmentation for the target customer, the CPU 121 reads the corresponding description from the health information 127 that is correlated with the segmentation, and provides the description to the target customer, for example, through the output I/F 124. The providing method is, for example, displaying on the display 52, the text message of an e-mail transmitted to the smartphone 38, or printing on a receipt to be received by the customer. The description relating to the health information of the target customer may be presented by presenting a message on an app executed on the smartphone 38.

Various aspects will be described below with reference to specific examples. In the following examples, it is assumed that the information processing device 100 is a PC and the health information 127 is displayed on the display 52 that is connected to the PC.

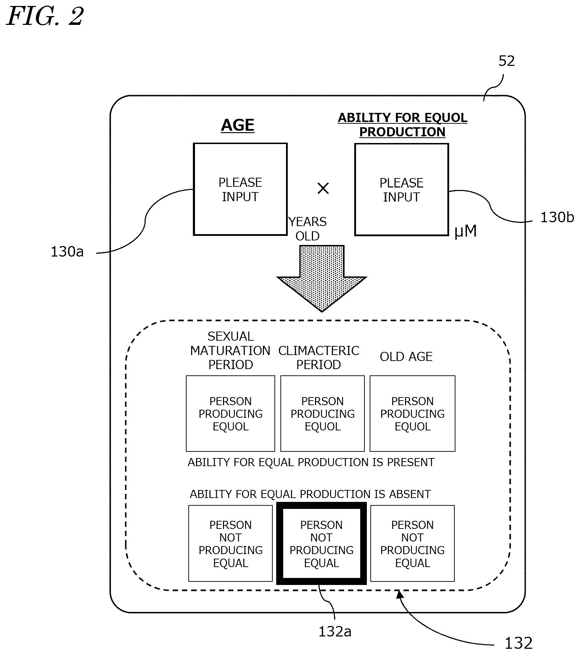

Fig. 2 is an example of a display used in the case where the health information 127 on the health of a woman is provided. A woman customer inputs the values indicating her age and her ability for equol production respectively into input cells 130a and 130b. The value indicating the ability for equol production can be acquired in advance by, for example, a urine examination.

The inventor set six segmentations 132 corresponding to the combinations each of the age and the ability for equol production. For example, the inventor divided the age into three types (life stages) that were a sexual maturation period, a climacteric period, and an old age, and further divided each of these into two types that were presence and absence of the ability for equol production to set the six segmentations 132 in total. The sexual maturation period, the climacteric period, and the old age correspond to three ranges divided in advance regarding the value of the age.

The CPU 121 determines which one of the sexual maturation period, the climacteric period, and the old age the age of the woman customer corresponds to, from the input value of the age, and further determines presence or absence of the ability for equol production that is input to determine which one of the segmentations the woman customer belongs to. For example, in the case where the age is 50 and the ability for equol production is absent, in the example in Fig. 2, a segmentation 132a is selected that has "non-producer in the climacteric period" attached thereto as a label. The age width corresponding to each of the sexual maturation period, the climacteric period, and the old age can be prepared in advance as a classification criterion. For example, the sexual maturation period is twenty years old or older and younger than forty-five years old, the climacteric period is forty-five years old or older and younger than fifty-five years old, and the old age is fifty-five years old or older.

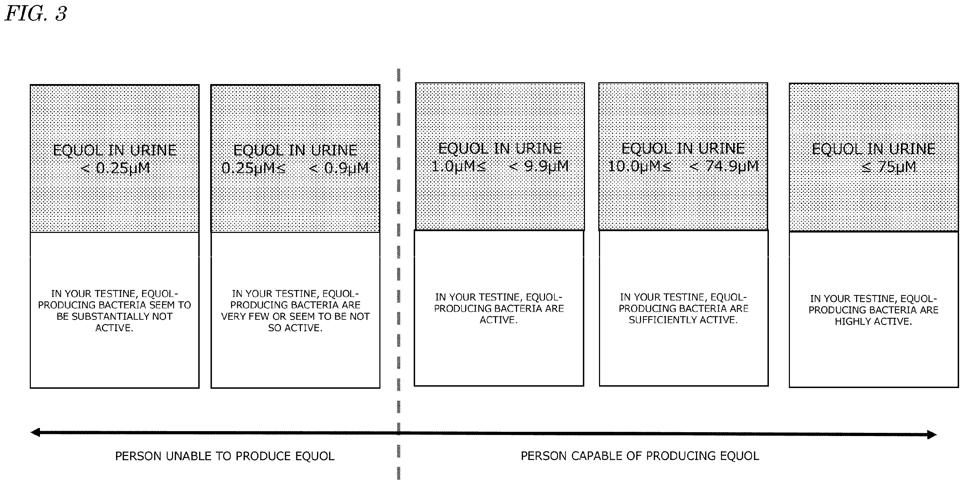

The presence or the absence of the ability for equol production can be determined based on the relation between the value indicating the ability for equol production and a specific threshold value (a classification criterion). In the case where the value of the ability for equol production is equal to or greater than the threshold value, the ability for equol production is determined as "present" and, in the case where the value is smaller than the threshold value, the ability for equol production is determined as "absent". For example, Fig. 3 shows an example of the classification criterion to classify the presence and the absence of the ability for equol production. In the case where equol in the urine is 1.0 µM or more, the ability for equol production is determined as "present" and, in the case where equol in the urine is 0.9 µM or less, the ability for equol production is determined as "absent". For example, 0.95 µM of equol in the urine can be set as the threshold value.

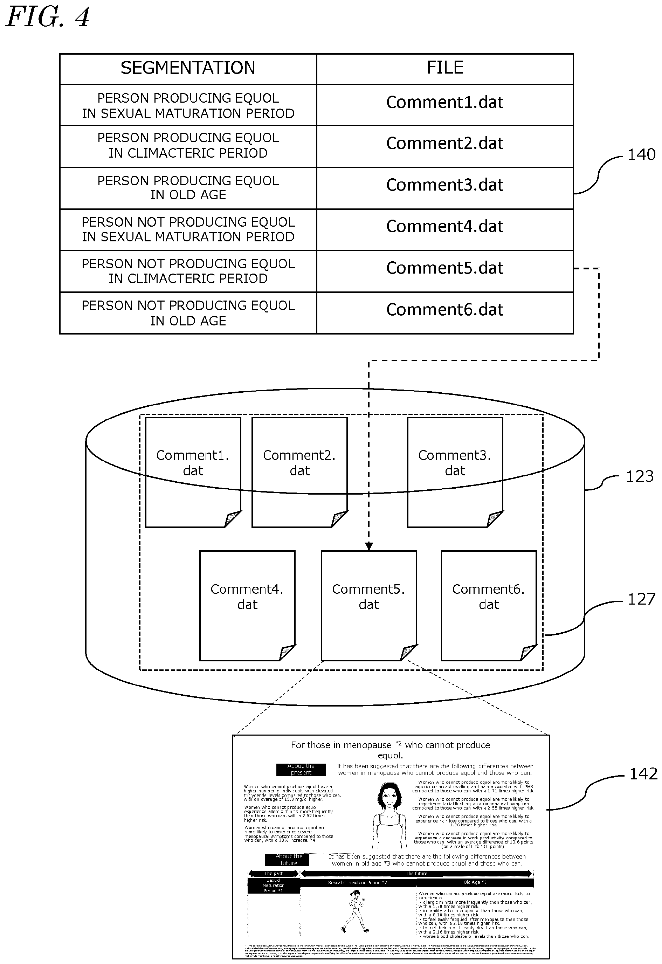

Fig. 4 shows a table 140 that correlates therein the segmentations relating to the health of a woman and file names with each other. The file names each indicate a file in which description corresponding to the health condition that corresponds to each of the segmentations is described. Fig. 4 shows that, in the case where the segmentation 132a of the "non-producer in the climacteric period" (Fig. 2) is selected, the segmentation 132a of the "non-producer in the climacteric period" is correlated with a file having a file name of "Commnt5.dat". The storage 123 has the file Commnet5.dat present therein and, in the file Comment5.dat, health information 142 on the woman who is in her climacteric period and who has no ability for equol production is described. In the table 140, a similar file name is correlated with each of the other five segmentations, and the file name indicates a file in which the description corresponding to the health condition of a woman who corresponds to one of the segmentations is described. In the description, it may be described that it is suggested, for example, that a woman incapable of producing equol tends to have a higher value of the neutral fat, has a higher risk of experiencing an allergic rhinitis, tends to have severer climacteric symptoms, and the like than those of a woman who can produce equol. Together with the above, products and health proposals to enhance the ability for equol production may be described. The overview of the segmentations other than the "non-producer in the climacteric period" will be described below.

The segmentation of a "producer in the sexual maturation period" targets a woman who is in her sexual maturation period and who has the ability for equol production. For example, a current difference can be considered as the description indicating the health condition, between a person corresponding to this segmentation and a woman who is in her sexual maturation period and who has no ability for equol production.

The segmentation of a "producer in the climacteric period" targets a woman who is in her climacteric period and who has the ability for equol production. For example, a current difference can be considered as the description indicating the health condition, between a person corresponding to this segmentation and a woman who is in her climacteric period and who has no ability for equol production.

The segmentation of a "producer in the old age" targets a woman who is in her old age and who has the ability for equol production. For example, a current difference can be considered as the description indicating the health condition, between a person corresponding to this segmentation and a woman who is in her old age and who has no ability for equol production.

The segmentation of a "non-producer in the sexual maturation period" targets a woman who is in her sexual maturation period and who has no ability for equol production. For example, a current difference can be considered as the description indicating the health condition, between a person corresponding to this segmentation and a woman who is in her sexual maturation period and who has the ability for equol production.

The segmentation of a "non-producer in the old age" targets a woman who is in her old age and who has no ability for equol production. For example, a current difference can be considered as the description indicating the health condition, between a person corresponding to this segmentation and a woman who is in her old age and who has the ability for equol production.

Such a difference can be considered as a further description indicating the health condition, as a difference in the future between a person corresponding to one of the segmentations and a woman who is in her climacteric period/old age and who has the ability for equol production, a difference in the future between a person corresponding to one of the segmentations and a woman who is in her climacteric period/old age and who has no ability for equol production, or the like.

Proposals may also be made for, for example, a service for using a fitness club or getting a massage, depending on the symptom. The table 140 correlates therein the merchandise and/or services to be proposed with the segmentations depending on the degree of the health condition. In the case, for example, where the information processing device 100 is used, the information processing device 100 can provide the prediction result and can propose the merchandise and/or services that correspond(s) to the result. This proposal can be similarly made in various examples described later.

According to the above process, even in the case where a change of the physical condition of the climacteric symptom does not yet become noticeable on the target customer, in other words, even for a potential climacteric symptom, the merchandise and/or the services can be proposed. In addition to the potential climacteric symptom, merchandise and/or services can similarly be proposed for a health problem or a premenstrual syndrome or a premenstrual dysphoric disorder (PMS, PMDD) of a woman in her old age.

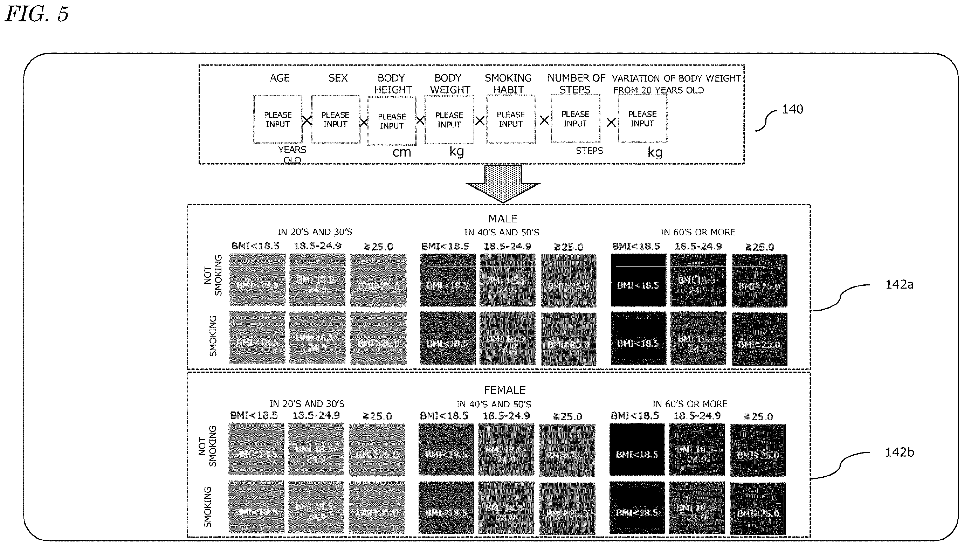

Fig. 5 is an example of a display used in the case where health information on the lifestyle is provided. In this example, the age, the sex, the body height, the body weight, presence or absence of a smoking habit, the number of steps, and a variation of the body weight in a specific time period are shown as the plurality of pieces of unique data 140 on the customer. Among these, information on, for example, the "presence or absence of a smoking habit" is the health data that reflects the health condition of the customer and the number of steps is the behavior data.

The inventor set many segmentations corresponding to the combinations each of the plurality of pieces of unique data 140. The inventor first set segmentation groups 142a and 142b corresponding to the sexes. The inventor next classified each of the segmentation groups 142a and 142b into three age groups based on the age. The inventor further classified each of the age groups into three sections based on the body-mass index (BMI) calculated from the body height and the body weight. The inventor further divided each of the sections into two types of presence and absence of a smoking habit. As a result, the inventor set eighteen segmentations in total for each of the segmentation groups 142a and 142b that correspond to the sexes. Similar to the examples in Fig. 2 and Fig. 4, in the example in Fig. 5, each of the segmentations is correlated with the health information prepared in advance.

When the customer inputs a plurality of pieces of unique data 140 on the customer, the CPU 121 determines the segmentation to which the customer belongs based on the plurality of pieces of unique data 140 and provides the health information that corresponds to the determined segmentation to the customer. For example, recommended number of steps per day can be presented as the health information. The customer can execute the health management for the customer using the health information.

According to the above process, for a potential health condition that causes a lifestyle-related disease in the target customer, the merchandise and/or services can also be proposed that improve or maintain the health condition. One or more selected from a change of the physical condition accompanied by a lifestyle-related disease and/or obesity, a lack of exercise, obesity, and a variation of the body weight in a specific time period can be targeted as the potential health condition.

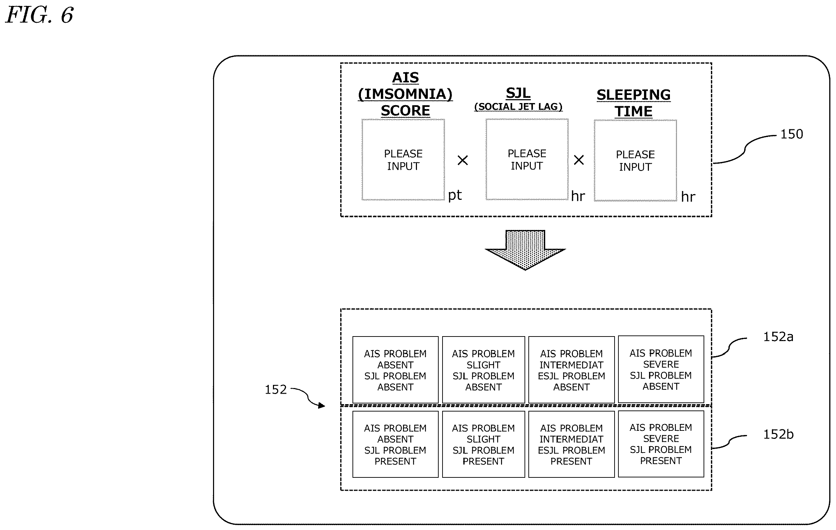

Fig. 6 is an example of a display used in the case where the health information on sleeping is provided. In this example, an insomnia (AIS) score, the time period value indicating the sleeping time, and the time period value indicating the social jet lag (SJL) are shown as the plurality of pieces of unique data 150 on the customer. Among these, for example, the time period value indicating the social jet lag is the symptom data that reflects the health condition of the customer. The "social jet lag" indicates the am unsteadiness of the rhythm in the body, and many studies point out that the health condition of each of the mind and the body is more influenced as the unsteadiness becomes severer, that is, the time period of the social jet lag is longer.

The inventor set eight segmentations 152 corresponding to the combinations each of the plurality of pieces of unique data 150. The inventor first divided the eight segmentations into a segmentation group 152a in the upper row and a segmentation groups 152b in the lower row corresponding to whether the social jet lag is shorter than one hour, or is one hour or longer. In Fig. 6, in the case where the social jet lag is one hour or shorter, an expression "a SJL problem is absent" is presented and, in the case where the social jet lag is longer than one hour, an expression "a SJL problem is present" is presented. For each of the segmentation group 152a and the segmentation group 152b, the degree of insomnia is classified into four stages corresponding to the insomnia score calculated using a predetermined method. For example, in this embodiment, using a scale for insomnia that is known as "Athens insomnia scale", the degree of insomnia is classified into the four stages that are "any insomnia problem is absent", "a slight problem is present", and "an intermediate and a severe problems are present". This indicates that, any problem is absent in the case where the insomnia score is smaller than 1, that is, 0, slight insomnia is present in the case where the insomnia score is 1 or greater and smaller than 4, moderate insomnia is present in the case where the score is 4 or greater and smaller than 6, and severe insomnia is present in the case where the insomnia score is 6 or greater. As a result, the inventor set the eight segmentations in total. Similar to the examples in Fig. 2 and Fig. 4, in the example in Fig. 6, each of the segmentations is correlated with health information prepared in advance.

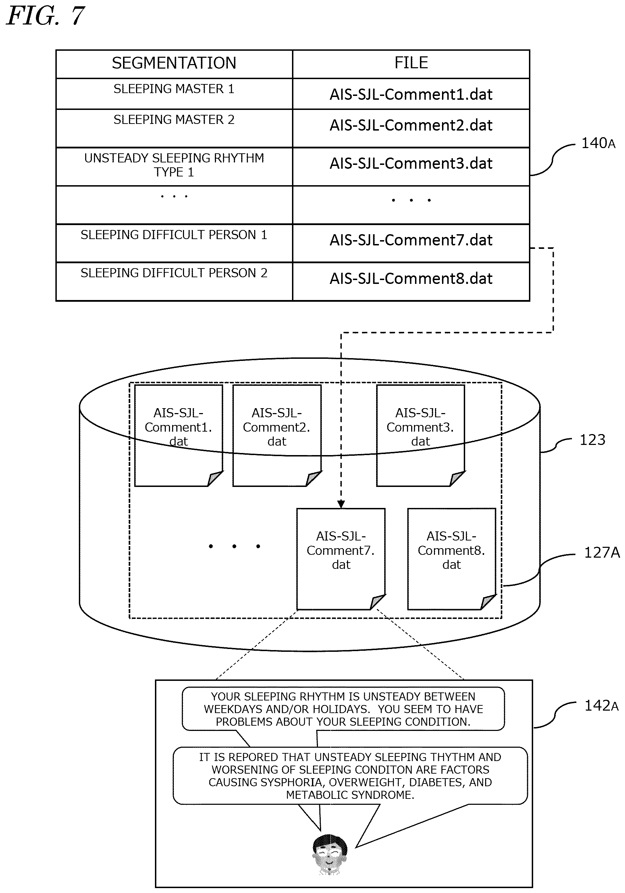

Fig. 7 shows a table 140A that correlates therein the segmentations relating to the sleeping and file names with each other. The file names each indicate a file in which description is described that corresponds to a health condition 127A corresponding to one of the segmentations. In Fig. 7, it is indicated that a file having a file name "AIS-SJL-Comment7.dat" is correlated with the segmentation of "sleeping difficult person 1". The file "AIS-SJL-Comment7.dat" is present in the storage 123 and, in the file "AIS-SJL-Comment7.dat", the health information 142A is described for the case where the SIS problem relating to insomnia is intermediate and the social jet lag SJL problem is present. In the health information 142A, for example, an illustration of a doctor and a words balloon for explanation are presented as if the doctor gives an explanation.

When the customer inputs a plurality of pieces of unique data 150 on the customer, the CPU 121 determines the segmentation to which the customer belongs based on the plurality of pieces of unique data 150 and provides the health information that corresponds to the determined segmentation to the customer. For example, necessity or unnecessity of consultation with a doctor, and the name of a drug to be preferably taken can be included in the health information. The customer can execute the health management for the customer using the health information.

The eight segmentations 152 set by the inventor based on the combinations each of the degree of the insomnia (AIS) problem, and the presence or absence of the social jet lag (SJL) problem will be described.

The degree of the AIS problem, and the presence or absence of the social jet lag (SJL) problem will hereinafter be represented by (AIS problem, SJL problem) = (absent, absent), or the like.
1:The case of (AIS problem, SJL problem) = (absent, absent) is classified into the segment of "sleeping master 1". This indicates, for example, that the customer can lead an orderly life on a regular basis and can therefore acquire excellent sleep. It is considered that maintaining this state is also effective for preventing a cold and improving the work performance, and this state is a preferred state.
2:The case of (AIS problem, SJL problem) = (slight, absent) is classified into the segment of "sleeping master 2". This indicates, for example, that the customer can lead an orderly life on a regular basis and can therefore acquire substantially excellent sleep. Because it is reported that a person has a risk of developing dysphoria when the person's sleeping condition becomes bad if any, it is preferred that the person corresponding to this segment be brought to attention.
3:The case of (AIS problem, SJL problem) = (absent, present) is classified into the segment of "unsteady sleeping rhythm type 1". The person corresponds to this segment has no problem on the person's sleeping while unsteadiness of the sleeping rhythm is observed between the weekdays and holidays. Because it is reported that the unsteadiness of the sleeping rhythm causes disorders of the mind and the body such as dysphoria, a metabolic syndrome, an overweight, and the like, the person corresponding to this segment only has to be brought to attention for the person to have an intention to improve the daily life rhythm from now.
4:The case of (AIS problem, SJL problem) = (slight, present) is classified into the segment of "unsteady sleeping rhythm type 2". It is assumed that the person corresponds to this segment has no problem on the person's sleeping while, because it is reported that a person has a risk of developing dysphoria when the person's sleeping condition is worsened if any, it is preferred that the person corresponding to this segment be brought to attention. Unsteadiness of the sleeping rhythm is observed between the weekdays and holidays. The same response as that in the above 3 is therefore preferable.
5:The case of (AIS problem, SJL problem) = (intermediate, absent) is classified into the segment of "sleeping situation to be noted type 1", and the person corresponding to this segment needs to pay attention to the person's sleeping situation. No unsteadiness of the sleeping rhythm is observed between the weekdays and holidays while the sleeping condition is worsened. It is reported that a person has a higher risk of developing dysphoria when the person's sleeping condition becomes worse. A person cannot acquire any excellent sleep when the person has any disorder in the person's mind or body. The person corresponding to this segment therefore only has to be brought to attention for the person to find the person's own methods of relaxing and refreshing not to accumulate any stress.
6:The case of (AIS problem, SJL problem) = (severe, absent) is classified into the segment of "sleeping situation to be noted type 2", and the person corresponds to this segment needs to pay attention to the person's sleeping situation. No unsteadiness of the sleeping rhythm is observed between the weekdays and holidays while the sleeping condition is worsened. It is reported that a person has a higher risk of developing dysphoria when the person's sleeping condition is worsened and, in addition, this situation also causes diabetes and a metabolic syndrome. A person cannot acquire any excellent sleep when the person has any disorder in the person's mind or body. The person corresponding to this segment therefore only has to be brought to attention for the person to find the person's own methods of relaxing and refreshing not to accumulate any stress.
7:The case of (AIS problem, SJL problem) = (intermediate, present) is classified into the segment of "sleeping difficult person 1". For the person corresponding to this segment, unsteadiness of the sleeping rhythm is observed between the weekdays and holidays, and it is estimated that the person has a problem on the person's sleeping condition. It is reported that unsteadiness of the sleeping rhythm and worsening of the sleeping condition are the factors of onset of dysphoria, an overweight, diabetes, and a metabolic syndrome. Various problems such as a disorder of a person's mind or body can be regarded as the cause for a person not to acquire any excellent sleep while it is considered that unsteadiness of the sleeping rhythm may be one cause of the above. The person corresponding to this segment only has to be brought to attention to live getting conscious about avoiding as much as possible the difference in the time of going to bed and/or that of getting up between weekdays and holidays.
8:The case of (AIS problem, SJL problem) = (severe, present) is classified into the segment of "sleeping difficult person 2". For the person corresponding to this segment, unsteadiness of the sleeping rhythm is observed between weekdays and holidays, and it is estimated that the person has a problem on the person's sleeping condition. It is reported that, when unsteadiness of the sleeping rhythm and significant worsening of the sleeping condition concurrently occur, a risk of developing each of dysphoria, an overweight, diabetes, and a metabolic syndrome becomes higher. Various problems such as a disorder of the mind or the body can be regarded as the cause for a person not to acquire any excellent sleep while it is considered that unsteadiness of the sleeping rhythm may be one cause of the above. The person corresponding to this segment only has to be brought to attention to avoid as much as possible the difference in the time of going to bed and/or that of getting up between weekdays and holidays.

In the above description, the unsteadiness of the rhythm in the body, more specifically, the sleeping rhythm of the customer is regarded as the potential health condition of the customer, and the health information on the potential health condition is proposed to the customer. In addition to the unsteadiness of the sleeping rhythm, for example, worsening of the sleeping condition and/or an improper sleeping time period may be regarded as the potential health condition of the customer. In this embodiment, one or more selected from a health problem caused by an unpreferable sleeping condition determined in advance, worsening of the sleeping condition, unsteadiness of the sleeping rhythm, and improper sleeping time period can be regarded as the potential health condition relating to the sleeping.

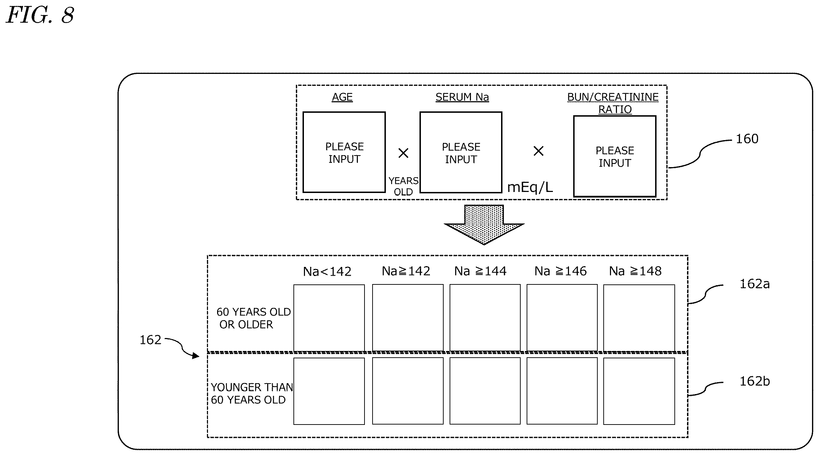

Fig. 8 is an example of a display used in the case where the health information on the water amount in the body is provided. In this example, the age, the serum Na value, and/or the BUN/creatinine ratio are presented as a plurality of pieces of unique data 160 on the customer. Among these, for example, the serum Na value and/or the BUN/creatinine ratio are/is the symptom data that reflects the health condition of the customer.

The inventor set 10 segmentations 162 corresponding to the combinations each of the plurality of pieces of unique data 160. The inventor first divided the segmentations into a segmentation group 162a in the upper row and a segmentation group 162b in the lower row corresponding to whether the age is 60 years old or older, or younger than that. For each of the segmentation groups 162a and 162b, the inventor next classified the range of the serum Na value in to five stages in the example in Fig. 8. For example, the five stages are the serum Na value<142, the serum Na value≥142, the serum Na value≥144, the serum Na value≥146, and the serum Na value≥148. As a result, the inventor set 10 segmentations in total. Similar to the examples in Fig. 2 and Fig. 4, in the example in Fig. 8, each of the segmentations is also correlated with the health information prepared in advance. The segmentation groups may each be classified corresponding to the range of the BUN/creatinine ratio, or the classification may be executed taking into consideration both of the serum Na value and the BUN/creatinine ratio.

When the customer inputs a plurality of pieces of unique data 160 on the customer, the CPU 121 determines the segmentation to which the customer belongs based on the plurality of pieces of unique data 160 and provides the health information that corresponds to the determined segmentation to the customer. For example, the degree of the problem on the water amount shortage, necessity or unnecessity of consultation with a doctor, and the name of a product to be preferably taken can be included each as the health information. The customer can execute the health management for the customer using the health information.

In the above example, the water amount shortage is evaluated using the serum Na value and the BUN/creatinine ratio. The water amount shortage can, however, be evaluated further using another index such as, for example, the urine specific gravity and/or the urine color. The serum Na value is a useful index to evaluate whether the customer has any chronic water amount shortage, and the urine specific gravity and the urine color are both useful indexes each to evaluate whether the customer has any fugitive water amount shortage. The diseases that the customer is easily affected with differ depending on whether the customer has any chronic water amount shortage or any fugitive water amount shortage.

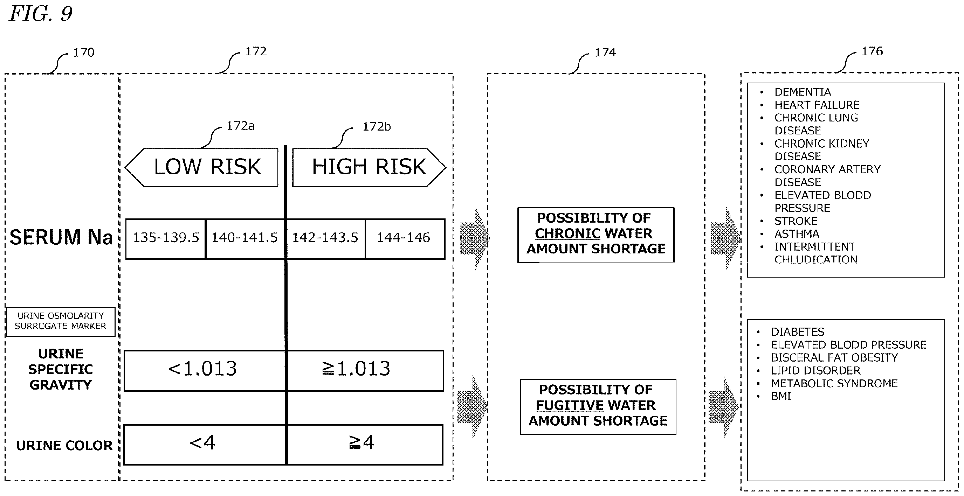

Fig. 9 shows a relation among segmentations 172 corresponding to input items, prediction results 174 as to whether the water amount shortage is present or absent, and predicted items 176 of the health risks caused by the water amount shortage, presented when the serum Na value, the urine specific gravity, and the urine color are input.

In Fig. 9, the evaluation is made that the customer belongs to a low risk segmentation 172a when the customers serum Na value is 135 or greater and smaller than 142, and the evaluation is made that customer belongs to a high risk segmentation 172b when the customer's serum Na value is 142 or greater and 146 or smaller. In the case where the evaluation is made that the customer belongs to the segmentation 172b based on the serum Na value, it is pointed out that the customer may have chronic water amount shortage. The "risk" as used herein means the health risk caused by the chronic water amount shortage and is, for example, dementia, a heart failure, a chronic lung disease, a chronic kidney disease, a coronary artery disease, an elevated blood pressure, a stroke, asthma, or intermittent claudication.

Similarly, evaluation is made for the low risk segmentation 172a when the customer's urine specific gravity is smaller than 1.013, and the evaluation is made for the high risk segmentation 172b when the urine specific gravity is 1.013 or greater. The urine color indicates the degree of the dehydration state. The urine color is determined by a doctor or the user by referring to a known color chart in which the urine color is divided into eight stages from no color or a faint color (the value: 1) to a dark color (the value: 8). For example, the evaluation is made for the low risk segmentation 172a when the urine color is lower than 4 (any of 1 to 3), and the evaluation is made for the high risk segmentation 172b when the urine color is 4 or higher (any of 4 to 8). In the case where the evaluation is made for the customer to belong to the segmentation 172b based on the urine specific gravity or the urine color, it is pointed out that the customer may have the fugitive water amount shortage. The "risk" relating to the urine specific gravity and the urine color means the health risk caused by the fugitive water amount shortage and is, for example, an increase of the rate of having each of such diseases as obesity, a high round-waist length, insulin resistance, diabetes, a low HDL, an elevated blood pressure, and a metabolic syndrome.

It is known that the serum Na value, the urine specific gravity, and the urine color each independently vary. The serum Na value presents a substantially constant value for each person. In the case where a person has no chronic water amount shortage, the person's serum Na value is smaller than 142 and, in the case where a person has chronic water amount shortage, the person's serum Na value is 142 or greater. In the case where a person having no chronic water amount shortage suffers from the fugitive water amount shortage, the person's serum Na value is still smaller than 142 (with the low risk) while, for example, the person's urine specific gravity can be 1.013 or greater (with the high risk) and the urine color can be 4 or greater. Whether the water amount shortage is chronic or fugitive can therefore be evaluated by combining the serum Na value, and the urine specific gravity and/or the urine color, and prevention of the health risk corresponding to the evaluation result can be executed.

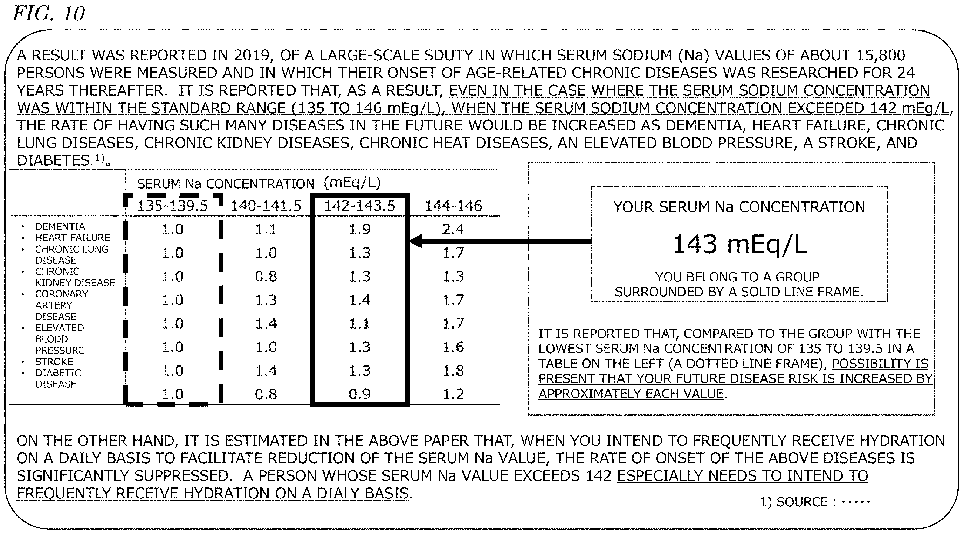

Fig. 10 is a first example of a display of the health information as to the health risk. This health information is presented to the customer who is evaluated to belong to the high risk segmentation from the serum Na value.

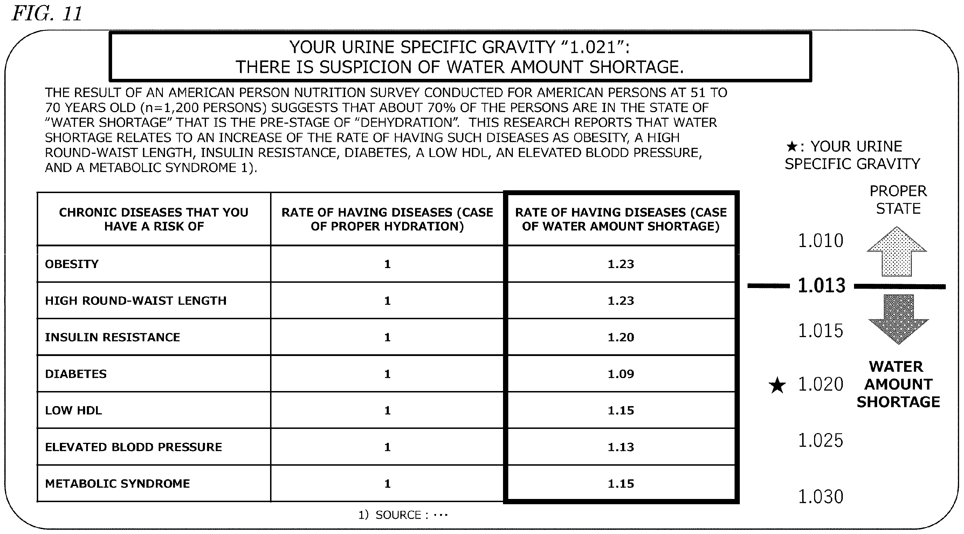

Fig. 11 is a second example of the display of the health information as to the health risk. This health information is presented to the customer who is evaluated to belong to the high risk segmentation from the urine specific gravity.

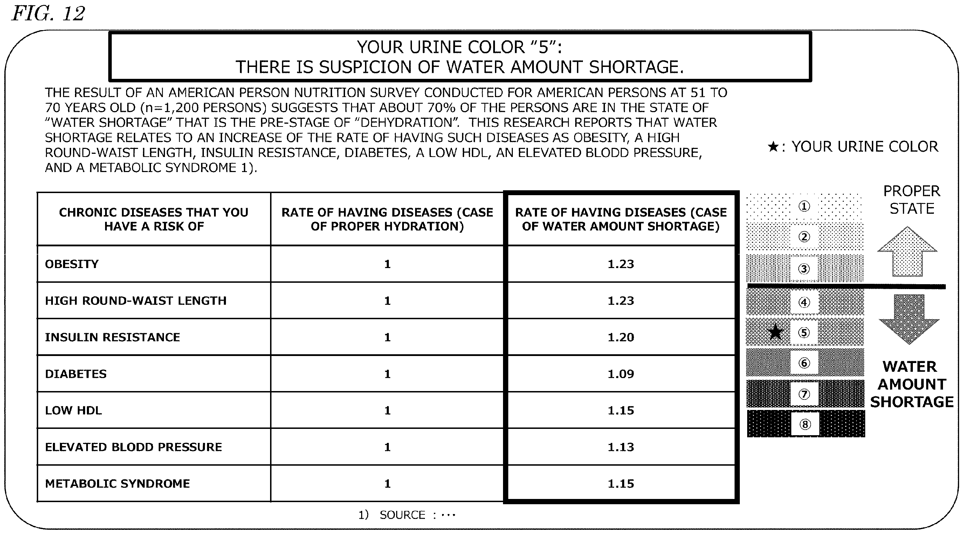

Fig. 12 is a third example of the display of the health information as to the health risk. This health information is presented to the customer who is evaluated to belong to the high risk segmentation from the urine color.

In the above description, the serum Na value alone, the serum Na value and the BUN/creatinine ratio, or the serum Na value, and the urine specific gravity and/or the urine color is/are regarded as the potential health condition of the customer, and the health information relating to the potential health condition is proposed to the customer. In addition to the serum Na value, for example, the BUN/creatinine ratio may be regarded as the potential health condition of the customer. In this embodiment, one or more selected from prediction of the health risk due to a chronic/fugitive water amount shortage, a high value of the serum Na, and/or a high value of the BUN/creatinine ratio can be employed as the potential health condition relating to the water amount in the body of the customer.

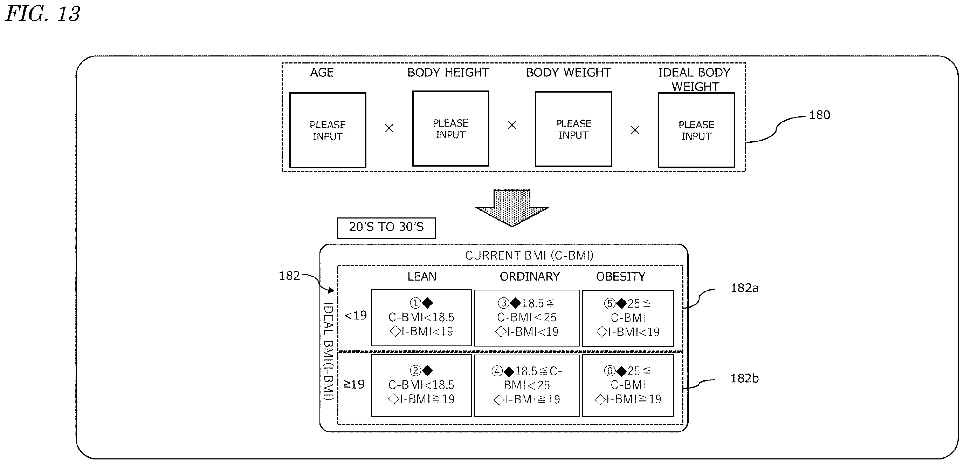

Fig. 13 is an example of a display used in the case where the health information on the nutrition is provided. In this example, the age, the body height, the body weight, and the body weight of a customer that the customer considers as ideal (the ideal body weight) are presented as a plurality of pieces of unique data 180 on the customer. Among these, for example, the age, the body height, and the body weight are symptom data that reflects the health condition of the customer.

The inventor set six segmentations 182 corresponding to the combinations of the plurality of pieces of unique data 180. The inventor first divided the six segmentations into a segmentation group 182a in the upper row and a segmentation group 182b in the lower row corresponding to whether the BMI that an individual person considers as ideal is smaller than 19, or equal to or greater than 19. The inventor next classified each of the segmentation groups 182a and 182b into three stages each determined relative to the current BMI in the example in Fig. 13. For example, the three stages were "lean", "ordinary", and "obesity". As a result, six segmentations 1 to 6 in total were set. Similar to the examples in Fig. 2 and Fig. 4, in the example in Fig. 13, each of the segmentations is correlated with health information prepared in advance.

When the customer inputs the plurality of pieces of unique data 160 on the customer, the CPU 121 determines the segmentation to which the customer belongs from the segmentations 1 to 6 based on the plurality of pieces of unique data 160, and provides the health information that corresponds to the determined segmentation to the customer.

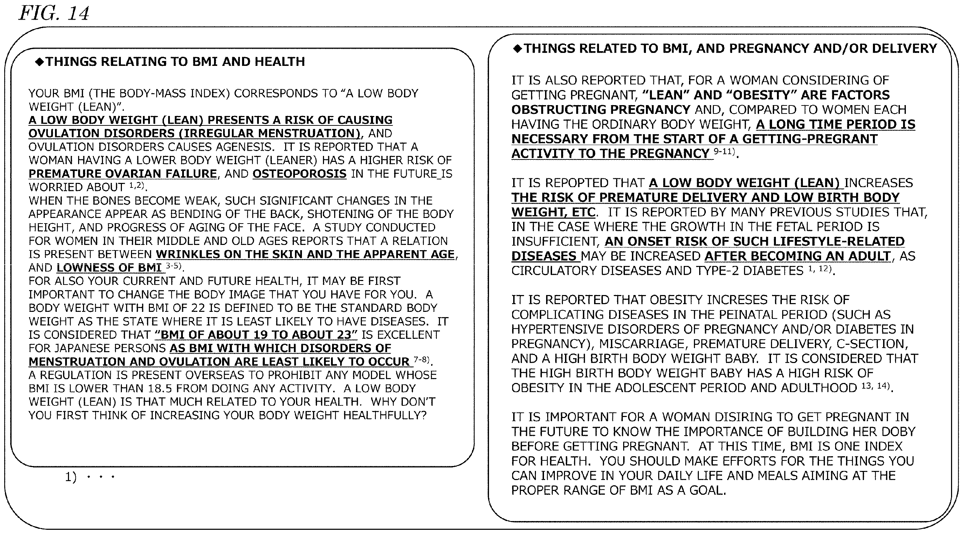

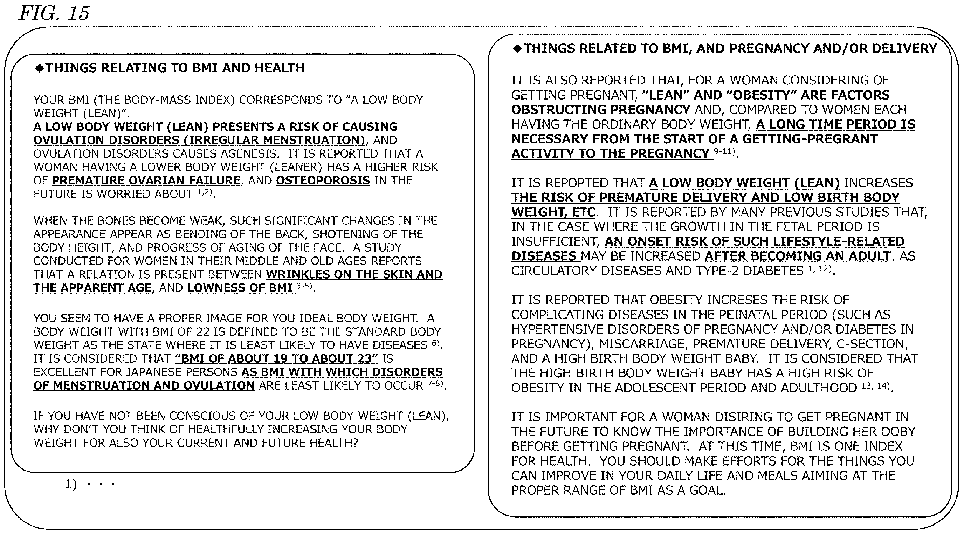

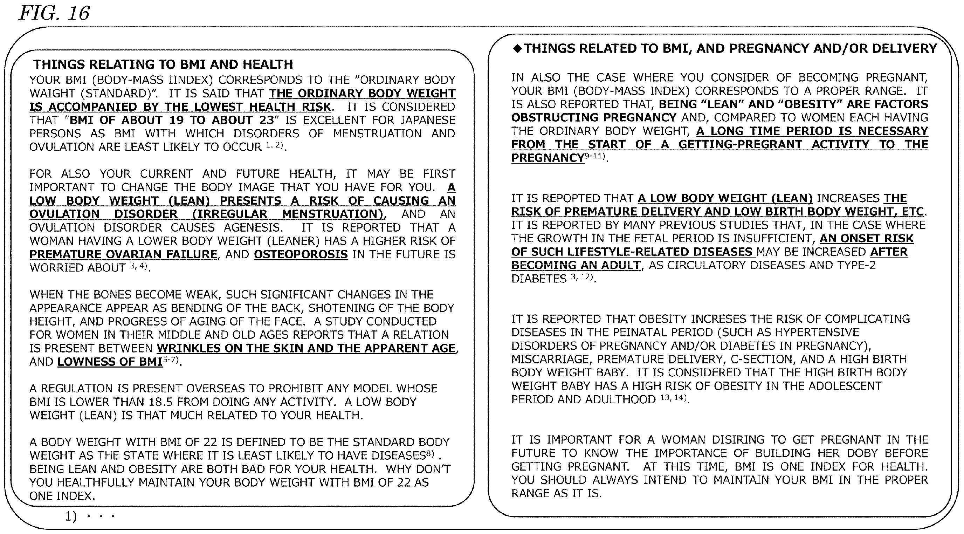

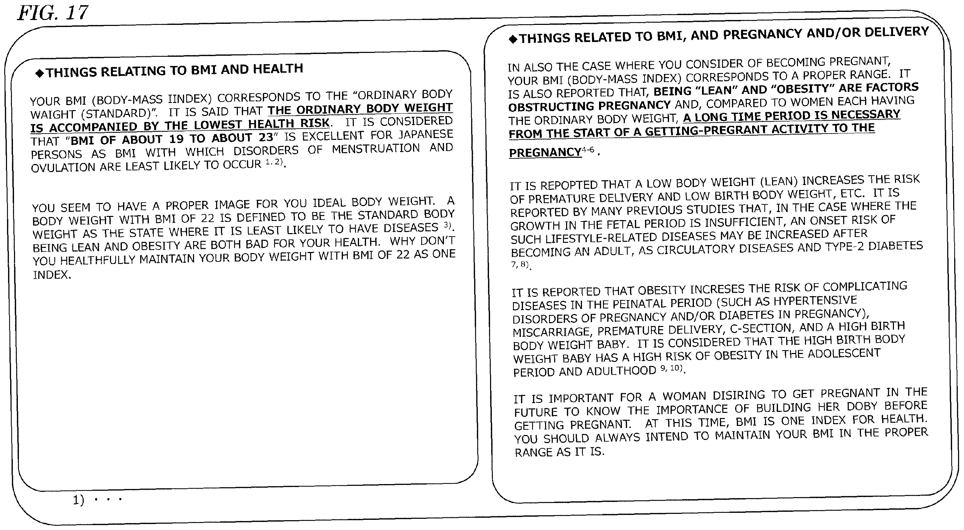

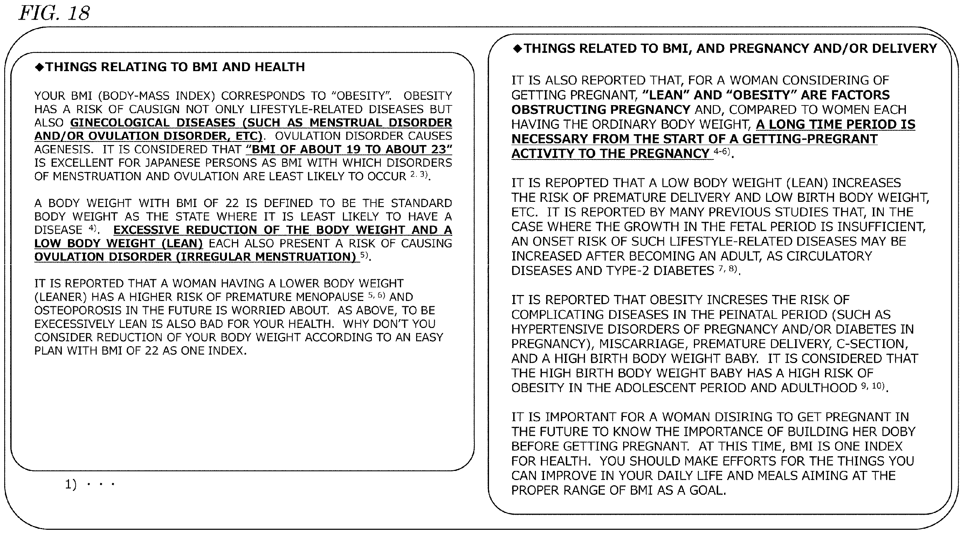

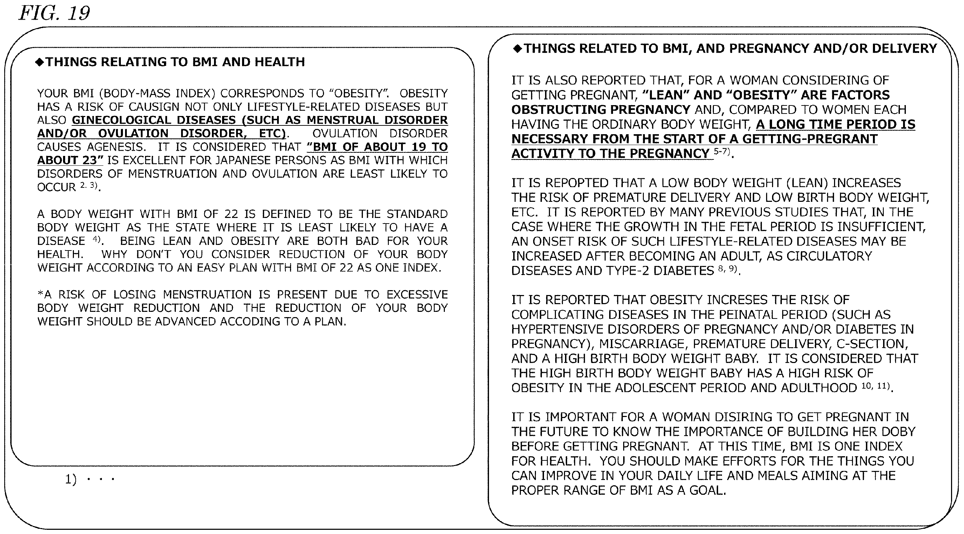

Fig. 14 to Fig. 19 show one example of the pieces of health information to be provided to the customer corresponding to the segmentations 1 to 6. In each of Fig. 14 to Fig. 19, the cell on the left presents the description for BMI and the health, and the cell on the right presents the description for BMI, and pregnancy and/or delivery. The description in the cell on the right may be provided to only those who desire to view. The description in each of the Fig. 14 to Fig. 19 is self-explanatory and will not therefore be specifically referred to. The content of the description in each of Fig. 14 to Fig. 19 is one example and a different description may be employed.

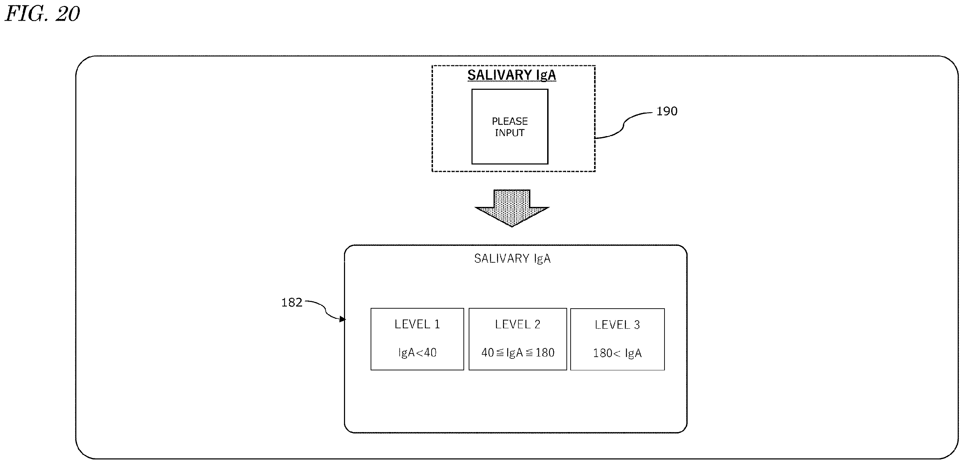

Fig. 20 shows a segmentation 182 that corresponds to the value of the salivary IgA, presented when the customer inputs the salivary IgA as a plurality of pieces of unique data 190 on the customer. In the example in Fig. 20, a segmentation group 182 is classified into three segmentations corresponding to the value of the salivary IgA. For example, the three segmentations are a segmentation of a level 1 with the salivary IgA<40, a segmentation of a level 2 with 40≤the salivary IgA≤180, and a segmentation of a level 3 with 180<the salivary IgA. In the example in Fig. 20, each of the segmentations is also correlated with the health information prepared in advance. The number of the segmentations may be two, or may be four or more.

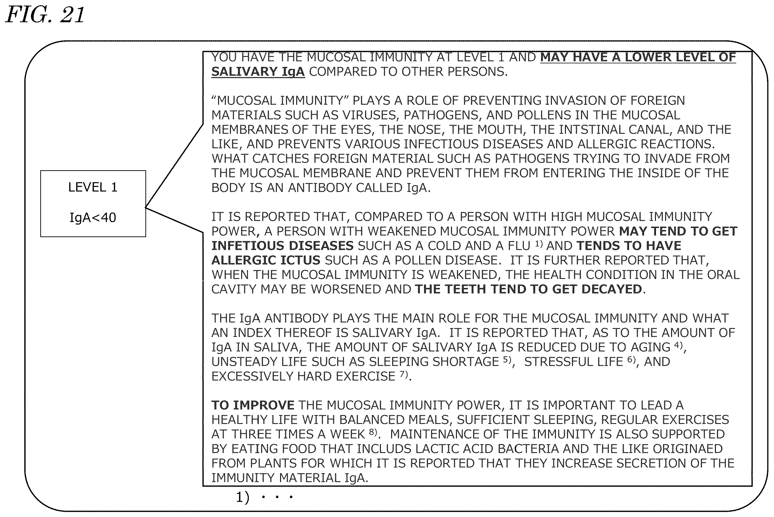

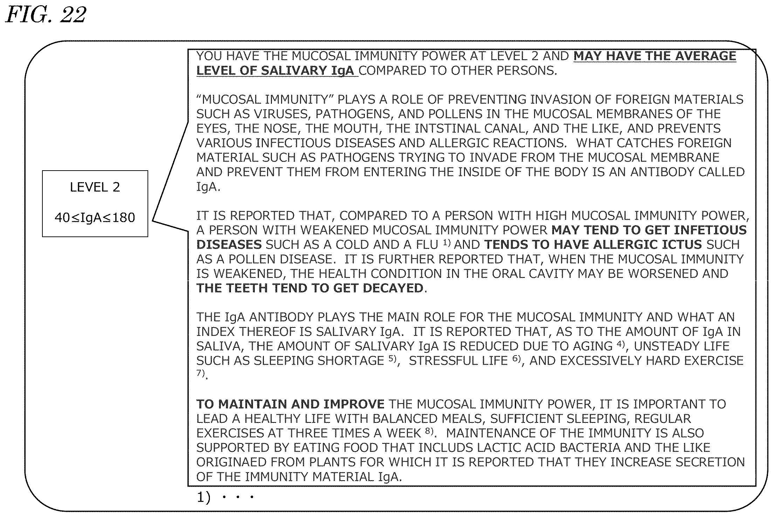

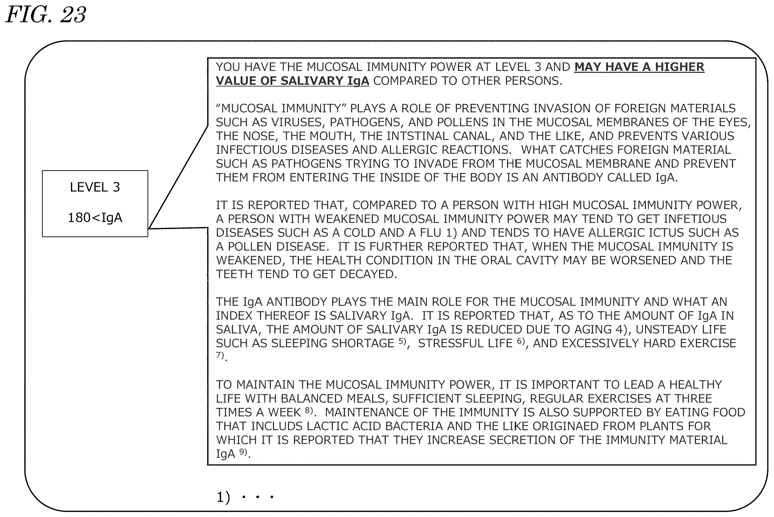

Fig. 21 to Fig. 23 show one example of the health information to be provided to the customer corresponding to the segmentations at the levels 1 to 3. In each of Fig. 21 to Fig. 23, the description is presented for each of the segmentations at the levels 1 to 3. The description in each of Fig. 21 to Fig. 23 is also self-explanatory and will not therefore be specifically referred to. The content of the description in each of Fig. 21 to Fig. 23 is one example and a different description may be employed.

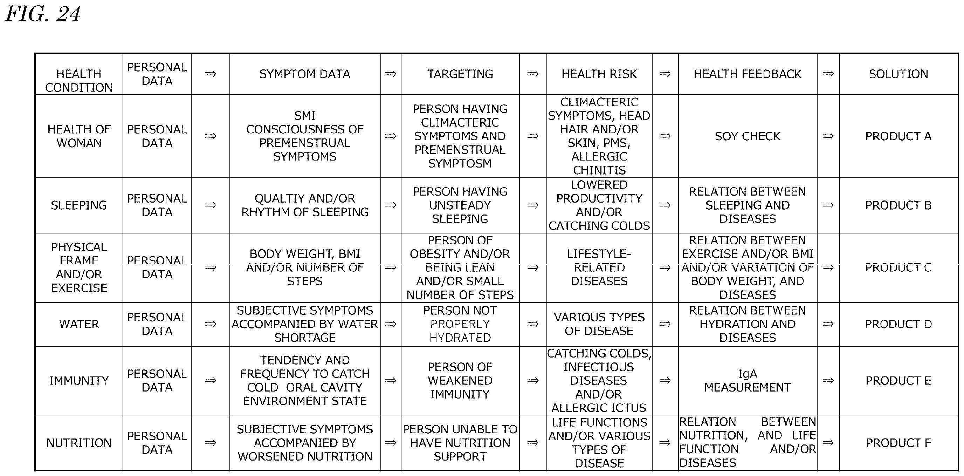

In this embodiment, description has been made in detail exemplifying the health of a woman, the sleeping, the exercise, the water, the nutrition, and the immunity as the health condition. For the exercise, the physical frame of each of the subjects can also be taken into consideration. The inventor considers that not only the description of each of the health conditions but also proposal for the products to further improve the health conditions are possible. Fig. 24 shows the details to execute health feedback and a proposal for products to improve the health condition, for each of various health conditions. As shown in Fig. 24, such solutions can be presented as, for example, proposal of a product E to a customer whose immune system is weakened, a proposal of a product F to a customer who lacks the nutrition state, and the like. For the customer whose immune system is weakened, one or more can be selected as the potential health information, from a health problem due to the weakened immune system, easiness to catch colds, an allergy symptom, and the condition of the oral cavity health, in addition to the salivary IgA. For the customer who lacks the nutrition state, one or more can be selected as the potential health information, from a health problem relating to the nutrition caused by the nutritious balance, poor nutrition, and the like, poor nutrition, a nutritional bias, and the dietary habit.

The above health conditions are only one example. The inventors of the present application make further studies on various health conditions, set a segmentation for each of the health conditions, and make explanations for each of the segmentations and extraction of the products to further improve each of the health conditions. Further other health conditions for which the studies were made will be listed as below.

Dementia, oxygen utilization, health of the blood vessels, oral cavity hygiene, health of the skin, health of the head hair, the intestinal environment, health of the mind and/ or depression and/or stress, the work engagement, health of the eyes, a headache, a back pain, shoulder stiffness, fatigue, health of the bones, health of the ears, health of the joints, the body temperature homeostasis (a feeling of cold), an increase in the appetite, climacteric disorders of a man, an elevated blood pressure (reduction of salt), a pain caused by the weather, and excessive sitting.

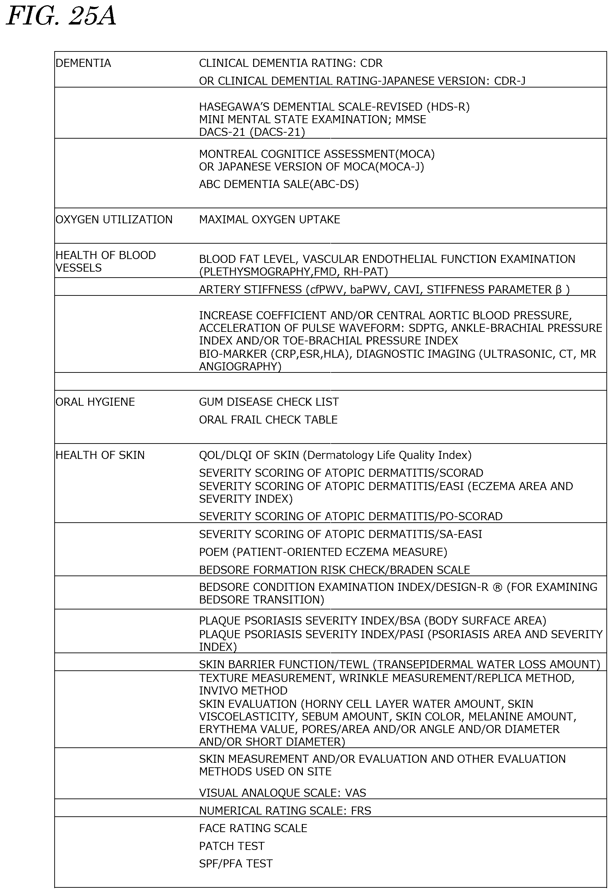

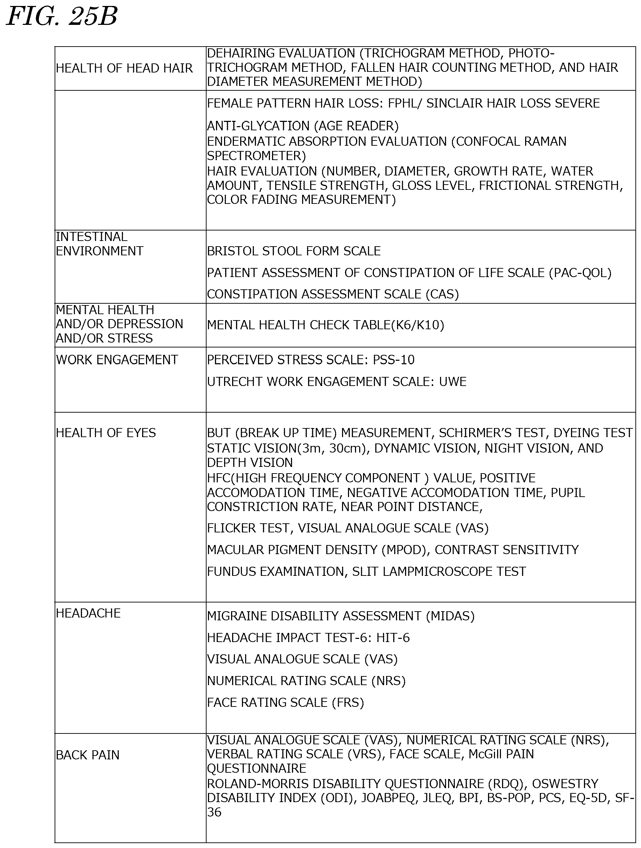

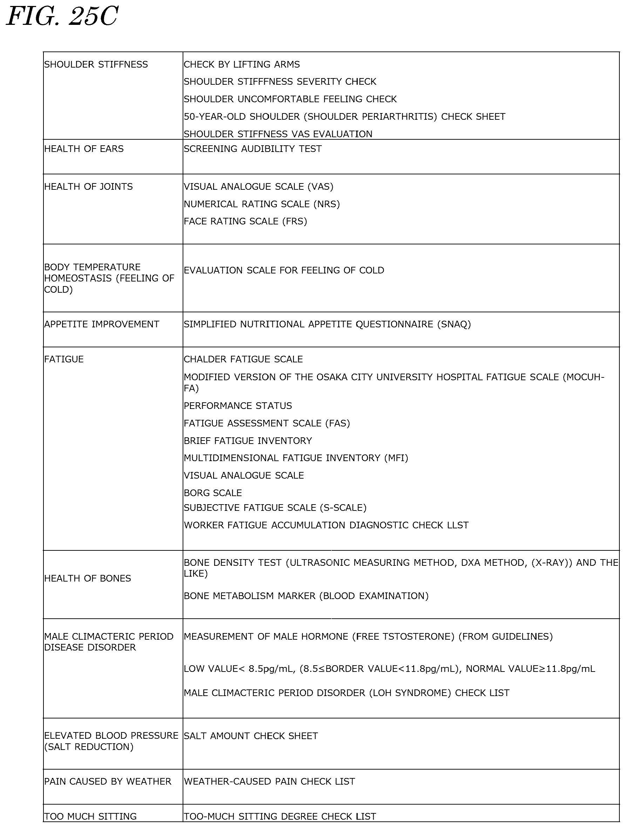

Fig. 25A to Fig. 25C show exemplification of further other health conditions and examples of the unique data to be input by the customer for each of the health conditions. Those skilled in the art can set one or plural threshold value(s) as necessary for the pieces of unique data and can set plural segmentations. Those skilled in the art cause the information processing device 100 to present the description of the health condition that corresponds to each of the segmentations and/or to present, to the customer, the description correlating therewith the products to further improve the health condition. It can be stated that the items to each be regarded as the health condition are countlessly present while the description on the health condition of the customer him/herself and the products to improve the health condition can be provided to the customer by employing the same mode of consideration for all the health conditions.

The proposal to improve the health symptom of a customer can be made by determining which segmentation of plural segmentations determined in advance the customer belongs to. For the potential health condition of the target customer, the proposal can thereby be also made, that avoids worsening of the health condition or that can improve the health condition to be better than the current one.

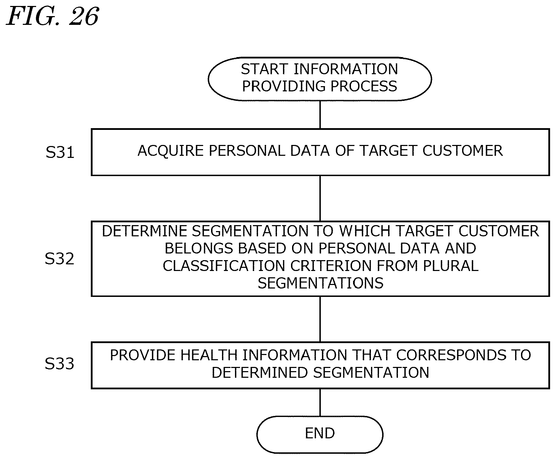

As above, the various aspects of the information provision using the information processing device 100 have been described. The aspects described so far are summarized in a process shown in Fig. 26.

Fig. 26 is a flowchart showing the procedure for a process according to this embodiment.

At step S31, the CPU 121 acquires the personal data of the target customer.

At step S32, the CPU 121 determines the segmentation to which the target customer belongs, from the plural segmentations based on the personal data and the classification criterion.

At step S33, the CPU 121 provides the health information that is correlated with the determined segmentation, corresponding to the determined segmentation.

The flowchart to be executed by the CPU 121 of the information processing device 100, described in the above embodiment can be realized as a computer program.

### (Second Embodiment)

An information providing system according to this embodiment provides various types of information on the health or provides an opportunity to receive a support from another person, to a customer or a potential customer who is a person of ordinary life. It is thereby enabled for the customer to continuously maintain and manage the health of the customer using the method that fits the customer, or for a product and/or an opportunity useful for the health of the customer to be continuously provided.

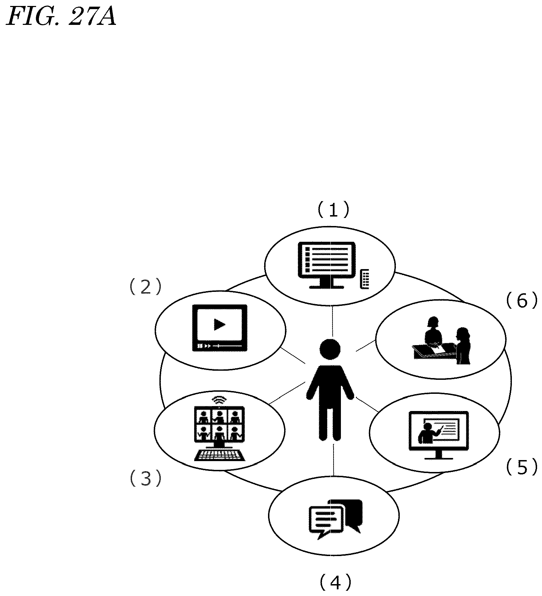

Fig. 27A schematically shows various types of information and opportunities received by the customer. As one example, the customer can acquire opportunities to receive the provision of the information and/or opportunities to receive supports, described in items (1) to (6). For example, the items are as follows. The number in parentheses is one example of an action from the information providing system to the customer.
Item (1) An opportunity to receive provision of the health information using a reading material (guiding to a health information site)
Item (2) An opportunity to receive provision of the health information using a moving picture (guiding to an action site)
Item (3) An opportunity to receive provision of the information using an on-line seminar (guiding to a seminar reservation site)
Item (4) An opportunity to receive a support by a consultation that uses e-mail or chat (starting up of e-mail or chat application software)
Item (5) An opportunity to receive an on-line support by a pharmaceutical chemist and/or a nutritionist (guiding to a reservation site)
Item (6) An opportunity to receive a face-to-face support by a pharmaceutical chemist and/or a nutritionist (guiding to a reservation site)

The above Items (1) and (2) are each an opportunity for the customer to be able to passively receive unilaterally the information. The above Item (3) is an opportunity for the customer to be able to actively receive bilaterally the information. The above Item (4) is an opportunity for the customer to be able to have a consultation one by one. The above Items (5) and (6) are opportunities for the customer to receive supports on-line and face to face from a pharmaceutical chemist and/or a nutritionist who are(is) expert(s). The customer can select an optional one from the above Items (1) to (6) through, for example, an information terminal owned by the customer such as, for example, a smartphone or a PC, and can utilize the information and the opportunity provided by the selected item. A link or a uniform resource location (URL) to transition from one certain item to another item may be disposed.

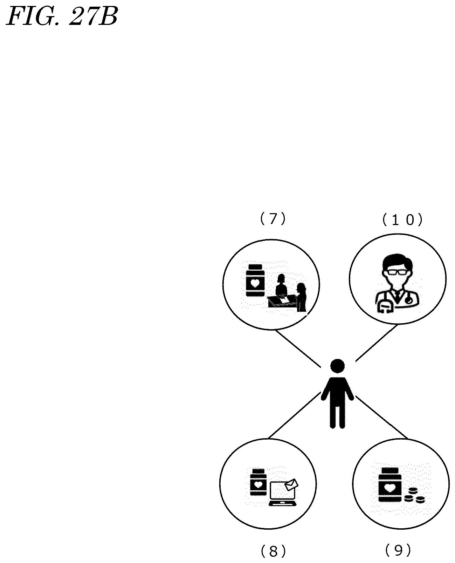

Fig. 27B shows one example of the services to be provided to the customer. For example, the services are as follows.
(7)Staff support
(8)Digital support
(9)Supplement
(10) Introduction to a doctor

Receiving the provision of the information and/or the opportunity shown in Fig. 27A through the customer's information terminal such as, for example, a smartphone or a PC, the customer clicks, for example, the link or the URL to receive any of the services (7) to (10) provided by the information providing system. The display on the display of the information terminal transitions to the display to receive any of the services (7) to (10). The customer is thereby enabled to receive the service needed by the customer.

The above opportunity can be variously present. It is, however, considered that in what aspect it is proper for the customer to receive the provision of the information and/or the service differs depending on the customer. For the customer, whether the provided information and/or opportunity fit(s) the customer influences the customer's desire to continuously receive the provision. It is therefore necessary for the information providing system to provide the information and/or the opportunity that is/are proper for each customer. An information providing system capable of providing the information and/or the opportunity that is/are proper for each customer will be described below.

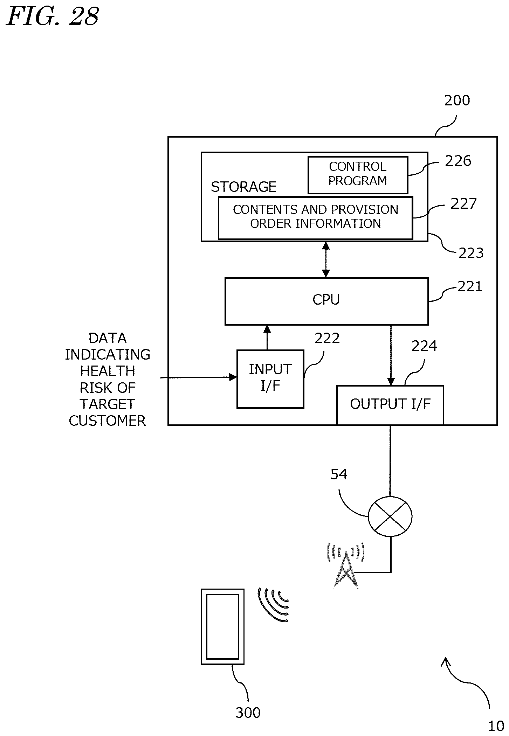

Fig. 28 is a block diagram showing the configuration of an information providing system 10 according to this embodiment.

The information providing system 10 includes a server device 200 and an information terminal 300.

The configuration of the server device 200 is substantially same as that of the information processing device 100 shown in Fig. 1. For example, the server device 200 can be, for example, a PC or a server computer. For example, the server device 200 includes a CPU 121, an input interface (I/F) 122, a storage 123, and an output interface (I/F) 124.

The CPU 121 is one example of the computing circuit of the server device 200 in this embodiment. The CPU 121 realizes the operation described later, by executing a control program 226 stored in the storage 223. The CPU 221 executes the control program 226, and the function as the server device 200 of this embodiment is thereby realized. The computing circuit constituted as the CPU 221 in this embodiment may be realized by one of various processors such as an MPU or a GPU.

The input I/F 222 is an input device to accept an input of risk data that indicates the degree of the health risk of the target customer. In this embodiment, the input I/F 222 is a communication terminal or a communication circuit that is connected to the communication network 54 or the like and that can thereby execute data communication therewith. The input I/F 222 receives the risk data that indicates the degree of the health risk of the customer transmitted by the customer from the information terminal 300. The risk data typically is the data that indicates the segmentation described in the first embodiment.

The storage 223 is a storage medium that stores therein the computer program 226 necessary for operating the server device 200, plural contents, and provision order information 227. The storage 223 can be, for example, a hard disc drive (HDD) or a solid state drive (SSD) that is a semiconductor storage device. The storage 223 may include a temporary storing element that includes a RAM such as, for example, a DRAM or an SRAM, or may function also as a working area for the CPU 221. The control program 226 is one example of the computer program in this embodiment. The details of the plural contents and the provision order information 227 will be described later.

The output I/F 224 is a communication circuit and/or a communication terminal that is(are) connected to various devices disposed outside the server device 200. For example, the output I/F 224 is a video output terminal connected to the display 52. The CPU 221 of the server device 200 transmits the information to be provided to the customer, to the information terminal 300 from the output I/F 224 through the communication network 54. As a result, the information is displayed on the display of the information terminal 300 owned by the customer, with the content and in the order that correspond to the customer. The customer can recognize the content displayed on the display of the information terminal 300, and can acquire the opportunity to receive the provision of the health information and the opportunity to receive the provision of the support from the experts or the like.

In the case where the input I/F 222 and the output I/F 224 are each the communication terminal or the communication circuit that is connected to the communication network 54 or the like and that can thereby execute the data communication, the hardware of each of the output I/F 224 and the input I/F 222 may be identical to each other. At this time, the input I/F 222 and the output I/F 224 can be collectively referred to as "communication interface (I/F)". The communication I/F is, for example, communication terminals of Ethernet (a registered trademark), or USB (a registered trademark) terminals. Otherwise, the communication I/F is communication circuits that each execute communication complying with a standard such as IEEE 802.11, 4G, or 5G. The communication I/F can be connected to a communication network such as an intranet or the Internet. The server device 200 may execute direct communication with another device through the communication I/F.

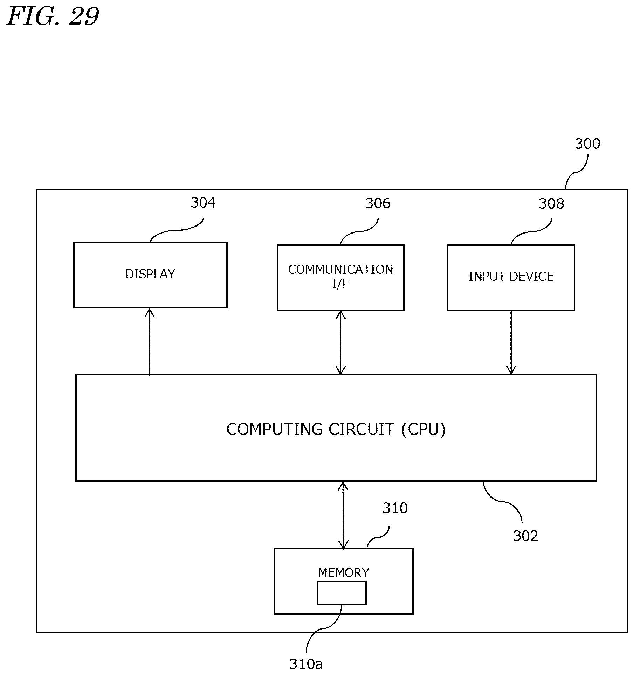

Fig. 29 is a block diagram showing the configuration of the information terminal 300. The information terminal 300 includes a computing circuit 302, a display 304, a communication interface (I/F) 306, an input device 308, and a memory 310.

The computing circuit 302 is a what-is-called semiconductor integrated circuit called "CPU". The CPU 302 is a computer mounted on the information terminal 300. The computing circuit 302 will hereinafter be referred to as "CPU 302".

The communication I/F 306 executes radio communication that complies with a communication standard such as, for example, 4 generation (4G) or 5G. The communication I/F 306 can also externally receive data through a communication network 1 and a radio base station.

The input device 308 is a device to operate the information terminal 300. The input device 308 is, for example, a touch screen panel or a hardware button disposed overlaid on the display 304. The display 304 is a displaying device that displays thereon the processing result by the CPU 302. The displaying device includes, for example, a liquid crystal displaying panel or an organic electro luminescence (EL) displaying panel.

The memory 310 stores therein a computer program 310a that is executed by the CPU 302. The memory 310 is herein a storage that encompasses a random access memory (RAM) and a read only memory (ROM). The computer program 310a stored in the ROM is read by the CPU 302 and is expanded in the RAM. The CPU 302 can thereby execute the computer program 310a. The operation of the information terminal 300 described below is realized by the computer program (an application program) 310a that is installed in the information terminal 300 to be executed thereby.

In this embodiment, the CPU 221 of the server device 200 transmits information to the information terminal 300 through the output I/F 224 and the communication network 54. The information terminal 300 displays the received information on the display 304 and thereby presents the received information to the customer.

A process executed mainly by the server device 200 of the information providing system 10 will be described below exemplifying the sleeping condition (the rhythm and the degree of the sleep loss) as "sleeping risk", as an example. The presence of the eight segmentations relating to the sleeping has been described in the example in Fig, 6. The sleeping risk will, however, be divided into three stages to be described below for convenience of the description. The three stages are "slight" for a slight sleeping risk, "intermediate" fir an intermediate sleeping risk, and "severe" for a severe sleeping risk. Which one of these segmentations the customer belongs to can be determined using the method described relating to the first embodiment.

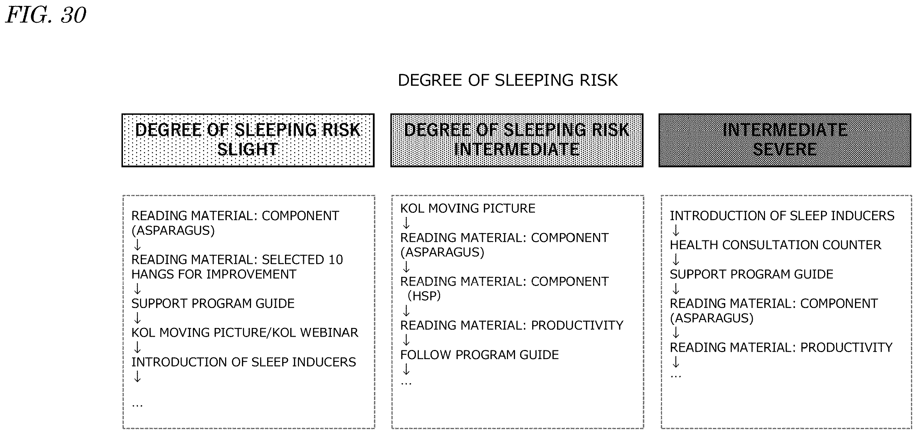

Fig. 30 shows an example where the CPU 221 of the server device 200 changes the information to be transmitted to the information terminal 300 depending on the degree of the sleeping risk. It is understood that, as shown in dotted lines in Fig. 30, the content and the order of the information displayed on the display of the information terminal are changed depending on the degree of the sleeping risk.

The CPU 221 of the server device 200 changes the information to be transmitted to the information terminal 300 depending on the degree of the sleeping risk of the customer and thereby changes the content and/or the order of the items to be displayed on the display 304 of the information terminal 300. For example, for a customer whose sleeping risk is "slight", the CPU 221 first transmits, to the information terminal 300, a URL of a webpage that carries a reading material on sleeping, and causes the display 304 to display thereon the reading material. The CPU 221 next transmits, to the information terminal 300, a URL of a webpage that carries a reading material describing a hang to improve the sleeping risk. Information to receive the provision of various services and/or various products that are useful for the customer whose sleeping risk is "slight" is thereafter also provided from the server device 200 to the information terminal 300. The reason why this display content is selected is that it is considered that the customer does not have so much knowledge about sleeping and it is therefore advantageous to first introduce a caring support program providing basic and specific information. An introduction moving picture and product information by a key opinion leader (KOL) such as a doctor, or an influencer, who influences the promotion of the product are presented later.

On the other hand, for a customer whose sleeping risk is "severe", the CPU 221 of the server device 200 first transmits a URL of a webpage that carries a reading material describing a product that is useful for reducing the sleeping risk. The CPU 221 next transmits a URL that guides to a reservation site of a health consultation counter. It is considered that this customer already acquires information and has an experience of trying by him/herself. Therefore, the fact that a product facilitating initiation of sleep is present is first introduced to facilitate purchasing the product, and the health consultation counter is introduced such that the customer can have a consultation with another person.

It is considered that a customer whose sleeping risk is "intermediate" knows the basic information. Therefore, for this customer, more detailed information such as a plus moving picture by a KOL and information on a component effective for sleeping is provided first, and information leading to purchase of the product is provided later.

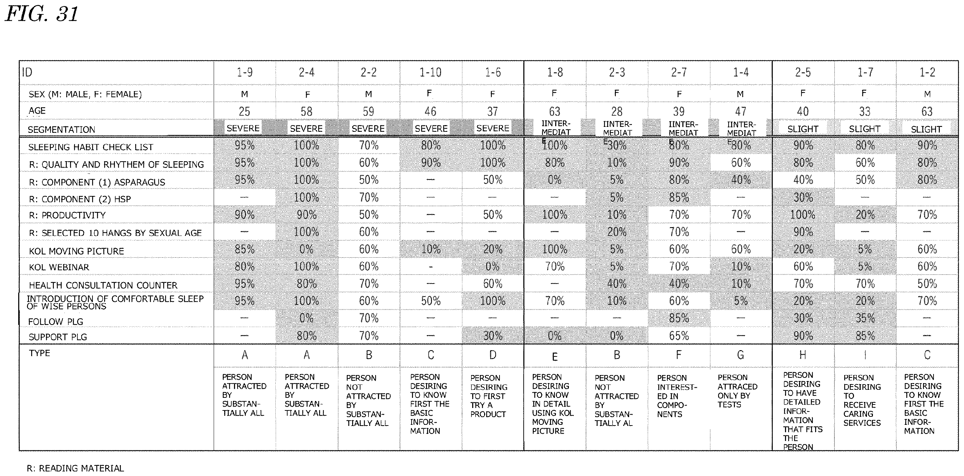

In what order the information is displayed corresponding on the degree of the sleeping risk of the customer may be determined by conducting in advance, for example, a questionnaire. Fig. 31 shows a table that summarizes the result of a questionnaire. The sleeping risk (the segmentation) of each of the persons who receive the questionnaire is first determined, various types of information are presented for each of the segmentations, and the persons each describe whether the content of the information is interesting and, more specifically, at what degree of the rate the content is interesting. The persons whose sleeping risks are equal are grouped (for "slight", "intermediate", and "severe") and the information only has to be provided in descending order of the degree at which the information is interesting in each of the groups.

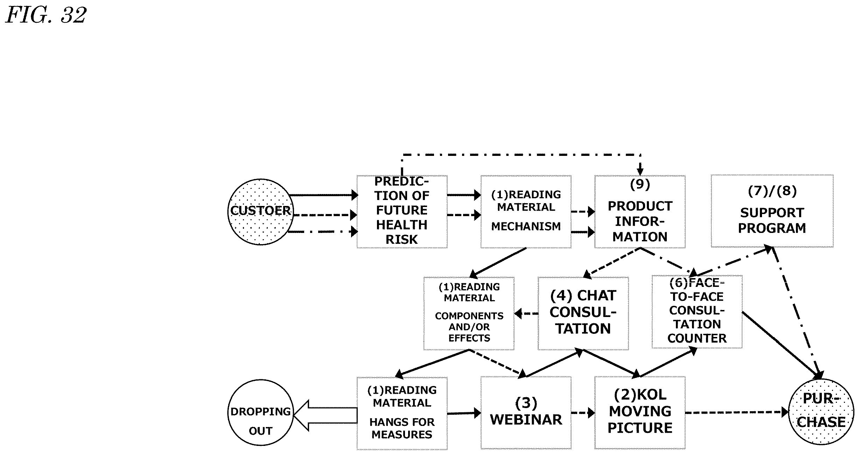

Fig. 32 is a transition diagram of the display content showing that the order of pieces of information to be provided is changed depending on "slight", "intermediate", or "severe" of the sleeping risk. A solid line indicates an example of the presentation to a customer in the segmentation of "slight", a dotted line indicates an example of the presentation to a customer in the segmentation of "intermediate", and a dashed-dotted line indicates an example of the presentation to a customer in the segmentation of "severe".

The storage 223 of the server device 200 stores therein, together with information on plural contents, provision order information that is the information defining the order of the provision of the information corresponding to the segmentation as shown in Fig. 30 or Fig. 32.

The above description is one example of the case where the degree of the sleeping risk is set as the segmentation. The other health risks only have to be similarly considered. The CPU 221 changes the information to be transmitted to the information terminal 300 depending on the degree of the sleeping risk (the segmentation) of the customer and thereby changes the contents and/or the order of the items to be displayed on the display 304 of the information terminal 300. The customer can thereby receive the provision of the information that is useful for the customer.

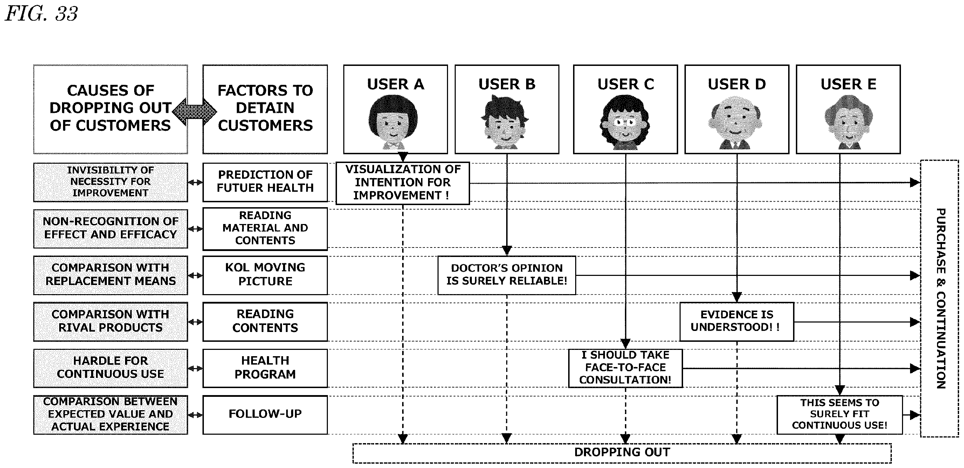

Fig. 33 is a diagram exemplifying the relation between the reason for the case where each of users A to E considers that the user desires to continuously purchase merchandise, and a factor influencing the reason (the presented information).

For example, the user B belonging to a certain segmentation views a KOL moving picture, considers that "it is a doctor's explanation and is surely reliable", and therefore determines to continuously purchase the merchandise. Measures can be taken to prevent each of the customers belonging to various segmentations from stopping purchasing, that is, from dropping out, by comprehensively preparing various contents.

For each of the health risks, plural contents and the provision order information 227 are provided in advance in the storage 223 of the server device 200. The CPU 221 of the server device 200 can thereby change the content to be displayed on the information terminal 300 and the order thereof for each of the types of the health risk of the customer depending on its degree (the segmentation).

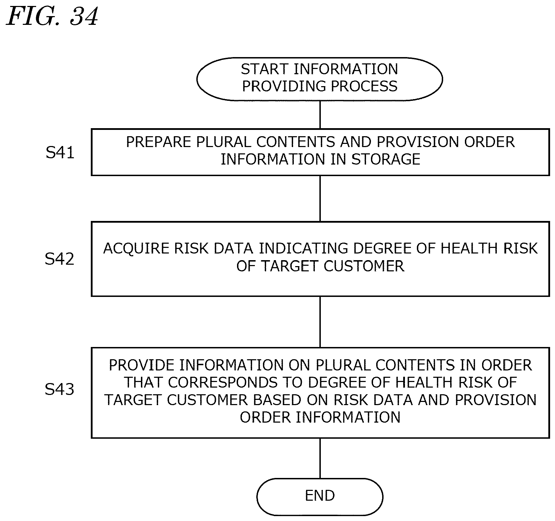

The operation of the server device 200 according to this embodiment is summarized in the process shown in Fig. 34.

Fig. 34 is a flowchart showing the procedure for the process according to this embodiment.

At step S41, the plural contents and the provision order information 227 are prepared to be stored in the storage 223.

At step S42, the CPU 221 acquires the risk data that indicates the degree of the health risk of the target customer through the input I/F 222.

At step S43, the CPU 221 provides the information on the plural contents in the order corresponding to the degree of the health risk of the target customer based on the risk data and the provision order information.

The above enables the presentation of the proper information to each of the customers.

### INDUSTRIAL APPLICABILITY

The exemplary embodiments of the present invention are usable for constructing a prediction model that is used to call attention for a subject, and for information provision to call attention therefor, to a predetermined disease, a disorder that does not correspond to a disease but that is accompanied by a symptom obstructing the subject's daily life, or the like.

### REFERENCE SIGNS LIST

- 100: information processing device
- 121: CPU (computing circuit)
- 122: input I/F
- 123: storage
- 124: output I/F

## Claims

1. An information providing method comprising:
acquiring personal data of a target customer;
determining a segmentation to which the target customer belongs, based on the personal data and a classification criterion that is prepared in advance, from a plurality of segmentations each indicating a class that corresponds to at least a degree of a health condition; and
providing health information that corresponds to the determined segmentation to the target customer,
the personal data comprising a plurality of pieces of unique data on the target customer,
at least one of the plurality of pieces of unique data being health data that reflects a health condition of the target customer,
the plurality of segmentations being classified corresponding to degrees of the health condition, and
each of the segmentations being correlated with health information prepared in advance corresponding to the health condition.

2. The information providing method according to claim 1, wherein
the degree of the health condition is classified in a plurality of numerical ranges,
each of the plurality of segmentations is correlated in advance with a numerical range that corresponds to the degree of the health condition,
a degree of a potential health condition of the target customer is indicated by a numerical value, and
the degree of the potential health condition of the target customer is predicted by determining the segmentation that corresponds to the numerical value, based on the numerical range to which the numerical value belongs.

3. The information providing method according to claim 2, wherein
the plurality of segmentations are three or more segmentations.

4. The information providing method according to claim 2 or 3, wherein
a table is prepared in advance, that correlates therein each of the segmentations, and a service and/or a product that suit(s) the health condition of each of the segmentations with each other, and
a proposal relating to the service and/or the product that suit(s) the health condition in the determined class, together with the health information, is further provided.

5. The information providing method according to claim 4, wherein
the health information and/or the proposal are/is provided through a text message in an e-mail, a display on a displaying device, printing on a receipt, or a message presented on an app executed by an electronic device.

6. The information providing method according to any one of claims 1 to 5, wherein
the potential health condition of the target customer is one or more selected from a health problem of a woman in her old age, a climacteric symptom, and a premenstrual syndrome or a premenstrual dysphoric disorder (PMS, PMDD) symptom.

7. The information providing method according to any one of claims 1 to 5, wherein
the potential health condition of the target customer is one or more selected from a health problem caused by an unfavorable sleeping condition determined in advance, worsening of a sleeping condition, unsteadiness of a sleep rhythm, and an improper sleeping time.

8. The information providing method according to any one of claims 1 to 5, wherein
the potential health condition of the target customer is one or more selected from a change of a physical condition and a lack of exercise accompanied by a lifestyle-related disease and/or obesity, obesity, and a variation of a body weight in a specific time period.

9. The information providing method according to any one of claims 1 to 5, wherein
the potential health information of the target customer is one or more selected from a health condition caused by a water shortage, a high value of serum Na, and a high value of a BUN/creatinine ratio.

10. The information providing method according to any one of claims 1 to 5, wherein
the potential health information of the target customer is one or more selected from a health problem caused by a weakened immunity, being easily catch colds, an SIgA concentration, an allergy symptom, and a condition of oral cavity health.

11. The information providing method according to any one of claims 1 to 5, wherein
the potential health information of the target customer is one or more selected from a health problem relating to nutrition, poor nutrition, a nutritional bias, and a dietary habit.

12. The information providing method according to claim 1, wherein
the target customer is a woman,
the plurality of pieces of unique data on the target customer comprise:
an age of the target customer; and
a value indicating an ability for equol production of the target customer as the health data.

13. The information providing method according to claim 12, wherein
the plurality of segmentations are classified corresponding to combinations of a plurality of life stages each determined corresponding to the age, and presence or absence of the ability for equol production, and
each of the segmentations is correlated with health information prepared in advance.

14. The information providing method according to claim 13, wherein
the presence or the absence of the ability for equol production is determined based on a relation between the value indicating the ability for equol production and a threshold value.

15. The information providing method according to claim 1, wherein
the plurality of pieces of unique data on the target customer comprise:
an age, a sex, a body height, a body weight, and a variation of the body weight in a specific time period, of the target customer;; and
behavior data relating to presence or absence of a smoking habit and number of steps of the target customer as the health data.

16. The information providing method according to claim 15, wherein
the plurality of segmentations are classified corresponding to combinations each of the age, the sex, a body-mass index, the variation of the body weight in a specific time period, and the behavior data on the number of steps, and the presence or the absence of the smoking habit, and
each of the segmentations is correlated with health information prepared in advance.

17. The information providing method according to claim 1, wherein
the plurality of pieces of unique data on the target customer comprise:
an insomnia score, and a time period value indicating the sleeping time, of the target customer; and
a time period value indicating a social jet lag of the target customer as the symptom data.

18. The information providing method according to claim 17, wherein
the plurality of segmentations are classified corresponding to combinations each of a degree of insomnia and the presence or the absence of the social jet lag, and
each of the segmentations is correlated with health information prepared in advance corresponding to a degree of a problem relating to sleeping as the health condition.

19. The information providing method according to claim 1, wherein
the plurality of pieces of unique data on the target customer comprise:
an age of the target customer; and
a serum Na value and a BUN/creatinine ratio of the target customer or
the serum Na value, and a urine specific gravity and/or a urine color of the target customer, as the health data.

20. The information providing method according to claim 19, wherein
the plurality of segmentations are classified corresponding to combinations each of the serum Na value and whether being equal to or older than, or younger than a predetermined age, and
each of the segmentations is correlated with health information prepared in advance corresponding to a degree of a problem relating to a water amount shortage as the health condition.

21. The information providing method according to 1, wherein
the plurality of pieces of unique data on the target customer comprise:
an ideal body weight that the target customer regards as being ideal; and
am age, a body height, and a body weight of the target customer, as the health data.

22. The information providing method according to claim 21, wherein
the plurality of segmentations are classified corresponding to combinations each of an age group determined corresponding to an age, a current body-mass index, and an ideal body-mass index calculated from the body height and the ideal body weight, and
each of the segmentations is correlated with health information prepared in advance corresponding to a degree of a problem relating to nutrition as the health condition.

23. The information providing method according to claim 1, wherein
the plurality of pieces of unique data on the target customer comprise salivary IgA.

24. The information providing method according to claim 23, wherein
the plurality of segmentations are classified corresponding to values of the salivary IgA, and
each of the segmentations is correlated with health information prepared in advance corresponding to a degree of a problem relating to an immunity as the health condition.

25. The information providing method according to any one of claims 1 to 24, wherein
the plurality of segmentations further comprise classes that each correspond to a daily behavior, and
the plurality of pieces of unique data comprised in the personal data further comprise health data that reflects a health condition of the target customer.

26. An information providing method comprising:
preparing in advance a plurality of contents to be presented to a customer, and provision order information that defines order of providing the plurality of contents corresponding to a degree of a health risk of the customer;
acquiring risk data that indicates a degree of a health risk of a target customer; and
providing the plurality of contents in order that corresponds to the degree of the health risk of the target customer based on the risk data and the provision order information.

27. The information providing method according to claim 26, wherein
the risk data is data indicating a segmentation selected from a plurality of segmentations each representing a class that corresponds to at least a degree of a health condition based on personal data of the target customer and a classification criterion that is prepared in advance,
the personal data comprises a plurality of pieces of unique data on the target customer,
at least one of the plurality of pieces of unique data is health data that reflects a health condition of the target customer,
the plurality of segmentations are classified corresponding to degrees of the health condition, and
each of the segmentations is correlated with health information prepared in advance corresponding to the health condition.
